# EUROPEAN PATENT SPECIFICATION

(11) **EP 3 797 776 B1**
(45) Date of publication and mention of the grant of the patent: **23.07.2025**
(21) Application number: 19200621.1
(22) Date of filing: 30.09.2019
(51) Int. Cl.: A61K 31/506, A61K 31/519, A61K 31/4545, A61K 31/5377, A61K 45/06, A61P 17/06, A61K 9/00, G01N 33/00

(54) **CDK4/6 INHIBITORS FOR THE TREATMENT OF PSORIASIS**
CDK4/6-HEMMER ZUR BEHANDLUNG VON PSORIASIS
INHIBITEURS DE CDK4/6 DESTINÉS AU TRAITEMENT DU PSORIASIS

(43) Date of publication of application: 31.03.2021
(73) Proprietor: Universitätsmedizin Mainz, 55131 Mainz (DE)
(72) Inventor: Kramer, Daniela, 72070 Tübingen (DE); Müller, Anne, 20099 Hamburg (DE); Schulze Osthoff, Klaus, 72070 Tübingen (DE)
(74) Representative: Witte, Weller & Partner Patentanwälte mbB

(56) References cited:
- EP-A1- 3 156 406
- WO-A1-2016/025650
- WO-A1-2020/023480
- CN-A- 109 876 000
- P. HENRI ET AL: "Psoriatic epidermis is associated with upregulation of CDK2 and inhibition of CDK4 activity", BRITISH JOURNAL OF DERMATOLOGY, vol. 182, no. 3, 25 August 2019 (2019-08-25), UK, pages 678 - 689, XP055680344, ISSN: 0007-0963, DOI: 10.1111/bjd.18178
- CLAUS JOHANSEN ET AL: "I[kappa]B[zeta] is a key driver in the development of psoriasis", PROCEEDINGS OF THE NATIONAL ACADEMY OF SCIENCES, vol. 112, no. 43, 12 October 2015 (2015-10-12), US, pages E5825 - E5833, XP055680178, ISSN: 0027-8424, DOI: 10.1073/pnas.1509971112
- ANNE MÜLLER ET AL: "I[kappa]B[zeta] is a key transcriptional regulator of IL-36-driven psoriasis-related gene expression in keratinocytes", PROCEEDINGS OF THE NATIONAL ACADEMY OF SCIENCES, vol. 115, no. 40, 17 September 2018 (2018-09-17), US, pages 10088 - 10093, XP055680180, ISSN: 0027-8424, DOI: 10.1073/pnas.1801377115

## Description

The present invention relates to the CDK4/6 inhibitor abemaciclib for use in the treatment of psoriasis via topical application on the skin, and a pharmaceutical composition for the treatment of psoriasis comprising said CDK4/6 inhibitor. The invention is defined by the claims.

Psoriasis is a long-lasting autoinflammatory skin disease, affecting 150 million individuals or 2-3% of the population worldwide. It is characterized by keratinocyte hyperproliferation and massive infiltration of immune cells into the skin, leading to the development of erythema, microabscesses, scales and increased skin thickness. The cause of psoriasis is not fully understood, however certain risk factors favor the development of psoriasis, including genetic predisposition, chronic infections, psychological factors such as stress, excessive alcohol consumption, cigarette smoking, and obesity etc. Importantly, psoriasis patients often develop several co-morbidities, such as arthritis, cardiovascular disease or diabetes, turning this autoinflammatory skin disorder into a life-threatening disease.

It is believed that psoriasis evolves from an abnormal activation of keratinocytes and dendritic cells, leading to the secretion of several cytokines, such as interleukin-36 (IL-36), interleukin-23 (IL-23) and interleukin-1β (IL-1β), which trigger the massive recruitment and activation of macrophages, neutrophils and T-cell subsets into the affected skin area. Most importantly, IL-17A-, IL-22- and TNFα-producing Th17 cells are recruited to the psoriatic lesions. Subsequently, these cytokines induce the hyperproliferation and dedifferentiation of keratinocytes, thereby further promoting the recruitment of immune cells and the development of chronic psoriatic skin lesions.

So far, no cure is available for psoriasis even though several treatment options do exist. Topical agents are typically used for mild disease, phototherapy for moderate disease, and systemic agents for severe disease. Topical agents include corticosteroid preparations, retinoids, vitamin D analogues, coal tar, moisturizers and emollients. Systemically applicable agents are essentially provided to suppress the immune system. They include include methotrexate and cyclosporin A. Further systemic treatments are based on the administration of hydroxycarbamide, fumarates and retinoids.

However, conventional medicinal psoriasis treatment is characterized by an unspecific mode of action of the administered agents. This non-specificity results in a low curative effect and eventually the development of adverse side effects.

Up-to-date therapies comprise several monoclonal antibodies (MABs) targeting cytokines or their cognate receptor, that promote disease progression. Theses antibodies are directed against IL-17 (i.e. secukinumab, ixekizumab and brodalumab), TNF-α (i.e infliximab, adalimumab and etanercept) or IL-23 (i.e. risankizumab, guselkumab and tildrakizumab). These neutralizing antibodies have been recently approved by the U.S. Food and Drug Administration (FDA) for treatment of psoriasis.

The systemic administration of neutralizing antibodies is associated with several disadvantages such as high costs, difficult application routes and systemic side effects such as upper respiratory tract infections, see Wasilewska et al., A new era in treatment of psoriasis and other skin diseases, Postepy Dermatol Alergol 33, 247-252 (2016). Importantly, therapeutic resistancies have been described based on the development of anti-drug antibodies (ADA) against the administered monoclonal antibodies.

Against this background the objective underlying the invention is to provide a novel kind of medical treatment of psoriasis where the disadvantages of the approaches in the art are avoided or at least reduced. In particular, such an agent should be provided which specifically addresses the molecular mechanisms underlying the development of psoriasis, thereby resulting in an improved curative effect while minimizing potential side effects.

This object is met by the provision of the CDK4/6 inhibitor abemaciclib for use in the treatment of psoriasis via topical application on the skin.

A "CDK4/6 inhibitor" according to the invention refers to any agent that specifically and inhibits the function of 'cyclin-dependent kinases' (CDK) 4 and 6. Examples of CDK4/6 inhibitors include abemaciclib (LY2835219), palbociclib (PD-0332991), ribociclib (LEE 011).

According to the invention "treatment" of psoriasis refers to the targeted reduction of a living being's symptoms of psoriasis.

While the invention allows the targeted treatment of psoriasis in any kind of living being, the treatment of a human being is preferred.

In an embodiment of the invention the CDK4/6 inhibitor may be used as the only active agent, however, in another embodiment it can also be used in combination with additional active agents against psoriasis.

The findings of the inventors were surprising and could not be expected, for the following reasons.

CDK4 and CDK6, in complex with cyclin D1, cyclin D2 or cyclin D3, represent well known cell-cycle regulating kinases that can phosphorylate RB, leading to the release of E2F transcription factors and G1-S cell cycle transition. Consistently, amplification of CDK4 and CDK6 as well as an overexpression of cyclin D proteins are frequently observed events in cancer, leading to the excessive proliferation of tumor cells. ATP-competitive CDK4/6 inhibitors, such as palbociclib and abemaciclib, have been developed for anticancer therapy and were recently approved for treatment of breast cancer patients; see Vidula, N. & Rugo, H.S. Cyclin-Dependent Kinase 4/6 Inhibitors for the Treatment of Breast Cancer: A Review of Preclinical and Clinical Data. Clin Breast Cancer 16, 8-17 (2016).

However, the state of the art does not provide any hints indicating that CDK4/6 inhibitor abemaciclib may have any beneficial effects in an inflammatory disease such psoriasis.

While documents US 9,616,062 and WO 2010/132725 fantasize an advantageous role of CDK4/6 inhibitors in psoriasis in a non-substituted manner and without the provision of any experimental evidences highly ranked scientific literature does not share this view. To the contrary, experts even point to the opposite direction and propose proinflammatory effects of CDK4/6 inhibitors.

For instance, Deng et al., CDK4/6 Inhibition Augments Antitumor Immunity by Enhancing T-cell Activation, Cancer Discov 8(2), 216-233 (2017), demonstrate by means of an experimental cancer model that the administration of CDK4/6 inhibitors such as palbociclib and trilaciclib (G1T28) results in increased levels of TH1 cytokines and tumor-infiltrating T cells.

Schaer et al., The CDK4/6 Inhibitor Abemaciclib Induces T Cell Inflamed Tumor Microenvironment and Enhances the Efficacy of PD-L1 Checkpoint Blockade, Cell Reports 22, 2978-2994 (2018), were able to demonstrate in murine syngeneic tumor models and *in vitro* assays that the administration of the CDK4/6 inhibitor abemaciclib results in an increased T cell activation and pro-inflammatory, intra-tumoral response.

WO 2016/025650 discloses pharmaceutical formulations comprising CDK4/6 inhibitor palbociclib (PD-332991) and an ERK inhibitor (Compound 1) which are used in experiments to demonstrate an effect on cell growth (Emax) in a number of cell lines which are all derived from cancerous epithelia in the lung or pancreas.

EP 3 156 406 discloses CDK4/6 inhibitor ribociclib, and pharmaceutical compositions comprising ribociclib, for use in the treatment of psoriasis.

CN 109 876 000 discloses topical preparations, such as ointments, powders, patches and sprays for the application of compositions comprising CDK4/6 inhibitor palbociclib. The treatment of psoriasis is not mentioned.

Against this background a person skilled in the art would have expected that the topical administration of CDK4/6 inhibitor abemaciclib on the skin of a living being affected by psoriasis would even encounter an amplification of psoriasis-related inflammation which is characteristic of said disease rather than achieving any beneficial or curing effect. The art teaches away from the invention.

Therefore, the skilled person would never have taken CDK4/6 inhibitor abemaciclib into consideration for the treatment of psoriasis.

The inventors have surprisingly realized for the very first time that the effect of the CDK4/6 inhibitors against psoriasis is not based on an inhibition of the cell cycle progression, but on the suppression of the cellular expression of the transcriptional regulator IκBζ. This is important because IκBζ, encoded by the gene *NFKBIZ,* has been identified as a transcriptional key regulator in psoriasis. IκBζ is commonly overexpressed in human psoriatic skin lesions; see Johansen, C., et al., I kappa B zeta is a key driver in the development of psoriasis, Proc Natl Acad Sci USA 112, E5825-E5833 (2015), and Müller, A., et al, IkappaBzeta is a key transcriptional regulator of IL-36-driven psoriasis-related gene expression in keratinocytes, Proc Natl Acad Sci USA 115, 10088-10093 (2018).

The invention is defined by the claims. The use of CDK4/6 inhibitor abemaciclib in the treatment of psoriasis as proposed by the invention thus addresses a molecular target that is specifically involved into the development of the disease. Thus, the invention allows a targeted treatment of psoriasis.

In addition, the cell cycle inhibitory activity of the CDK4/6 inhibitors is of particular advantage, since it results in an additional blocking of the keratinocyte hyperproliferation which is a hallmark of psoriasis.

Furthermore, the inventors have realized that genes which are not IκBζ-dependent remain unaffected upon CDK4/6 inhibition. These findings indicate that the inhibition of IκBζ in psoriasis treatment will be rather specific and not associated with side effects, which are commonly found upon a broad inhibition of NF-κB, e.g. by toxic IKK inhibitors.

Even more importantly, as the inhibition of IκBζ by CDK4/6 inhibitors blocks multiple pathways in psoriasis, i.e. IL-17, IL-23, and IL-36 signalling, targeting IκBζ might increase overall therapeutic responses as well as prevent the development of therapy resistance.

In the invention the use of the CDK4/6 inhibitor in the treatment of psoriasis is carried out via a topical application on the skin.

Importantly, the inventors were able to demonstrate via two different experimental models that a topical administration of CDK4/6 inhibitors to the skin provides effective protection against psoriasis. This measure has the advantage that the treatment of psoriasis is focused on the site of the pathologic events. Any potential side effects can be further reduced herewith. The topical administration onto the skin is less expensive than a systemic application and can be easily realized by the patient. Furthermore, the risk of the development of a therapy resistance is significantly reduced.

In another embodiment of the invention the CDK4/6 inhibitor is provided as a skin-permeable formulation.

The inventors succeeded in delivering CDK4/6 inhibitors of hydrophobic nature, exemplified by ademaciclib, into the deeper layers of the skin of an experimental animal, thereby completely suppressing psoriasis-typical skin inflammation. This measure has, therefore, the advantage, that the CDK4/6 inhibitor is provided in a safe form allowing the targeting treatment of psoriasis while, at the same time, minimizing any potential side effects.

In a further embodiment of the invention the skin-permeable formulation is selected from the group consisting of: crème, gel, lotion.

This measure has the advantage that the CDK4/6 inhibitor is provided in a well-established formulation variant that allows the targeted administration onto the skin.

The CDK4/6 inhibitor is an ATP-competitive CDK4/6 inhibitor, namely abemaciclib.

This measure has the virtue that such kind of CDK4/6 inhibitor is deployed which has been tested in the treatment of cancer diseases such as breast cancer. There this agent is well tolerized with nearly no adverse effects. The invention therefore takes advantage of the long-term experience made with cancer treatment by such compound. Thus, by this embodiment the provision of a particularly suited agent is ensured.

"Abemaciclib" [CAS 1231929-97-7 (Free Base); 1231930-82-7 (Mesylate Salt)], also referred to as LY2835219, Verzenio or Verzenios was originally developed for the treatment of advanced breast cancer. It was approved in the United States by the FDA on 28 September 2017 for "adult patients who have hormone receptor (HR)-positive, human epidermal growth factor receptor 2 (HER2)-negative advanced or metastatic breast cancer that has progressed after taking therapy that alters a patient's hormones.

"Palbociclib" (CAS 571190-30-2), not claimed, also referred to as PD-0332991, Ibrance or Palbonix, was developed for the treatment of HR-positive and HER2-negative breast cancer. The drug was approved by the FDA in an accelerated program on 3 February 2015 as the first CDK4/6 inhibitor for a treatment in combination with letrozole for patients with estrogen receptor positive (ER+) advanced breast cancer. Approvals in other countries followed.

"Ribociclib" (CAS 1211441-98-3), not claimed, also named LEE-011 or Kisqali was also developed for certain types of breast cancer. It was approved in the United States by the FDA in March 2017 and the European Medicines Agency in August 2017 for use in combination with an aromatase inhibitor such as letrozole to treat HR-positive, HER2-negative advanced or metastatic breast cancers.

"Trilaciclib" (CAS 1374743-00-6), not claimed, also referred to as G1T28 is a small molecule, competitive inhibitor of CDK4/6, with potential antineoplastic and chemoprotective activities. It is crrently undergoing clinical trials. In August 2019 it has received 'breakthrough therapy' status for small cell lung cancer in the USA.

In another embodiment of the invention the CDK4/6 inhibitor is used in combination with an additional agent active against psoriasis-associated symptoms.

Such measure may result in an even stronger effectiveness of the invention in the treatment of psoriasis. In particular, due to the combination of the CDK4/6 inhibitor and the additional agent synergistic effects may come into play. According to this embodiment of the invention, any additional agent active against psoriasis-associated symptoms is, in principle, suitable. Examples of such additional active agents include corticosteroids, retinoids, vitamin D analogues, coal tar, moisturizers and emollients, etc.

In a further embodiment of the invention the additional agent is an EZH2 inhibitor.

The inventors have surprisingly realized that EZH2 inhibitors are likewise capable of treating psoriasis. Examples of EZH2 inhibitors include EPZ-6438 (tazemetostat), CPI-169, 3-deazaneplanocin A (DZNep), EPZ005687, El1, GSK126, UNC1999, CPI-1205, EPZ011989, EBI-2511, PF-06726304, GSK503, and GSK343. The combination of both active agents may therefore lead to a substantive action amplification resulting in an even more efficient treatment.

Another subject-matter of the invention relates to a pharmaceutical composition for the treatment of psoriasis comprising the CDK4/6 inhibitor according to the invention and a pharmaceutically acceptable carrier.

Pharmaceutically acceptable carriers are well known to the skilled person. They allow a proper formulation of the active agent and serve to improve the selectivity, effectiveness, and/or safety of drug administration. Pharmaceutically acceptable carriers include, without being limited thereto, solvents, fillers, binders, lubricants, stabilizers, surfactants, suspensions, thickeners, emulsifiers, preserving agents, liposomes, micelles, microspheres, nanoparticles, etc. suitable for the particular form of dosage. Except for cases, when the medium of conventional carriers is incompatible with the active ingredient, for example, upon occurrence of any undesirable biological effects or other adverse interactions with any other ingredient(s) of the pharmaceutical composition, the use of such compositions falls within the scope of this invention, as defined in the claims. Materials that can serve as pharmaceutically acceptable carriers include, but are not limited to, monosaccharides and oligosaccharides, as well as derivatives thereof; malt, gelatin; talc; excipients such as: cocoa butter and suppository waxes; oils, such as peanut oil, cottonseed oil, safflower oil, sesame oil, olive oil, corn oil and soybean oil; glycols such as propylene glycol; esters, such as ethyl oleate and ethyl laurate; agar; buffering agents, such as magnesium hydroxide and aluminium hydroxide; alginic acid; pyrogen-free water; isotonic saline, Ringer's solution; ethyl alcohol and phosphate buffer solutions. In addition, the composition may contain other non-toxic compatible lubricants, for example sodium lauryl sulfate and magnesium stearate, as well as coloring agents, parting liquids, film formers, sweeteners, flavoring additives and flavorants, preserving agents and antioxidants.

In an embodiment of the invention the pharmaceutical preparation can include the CDK4/6 inhibitor as the only active agent, however, in another embodiment it can also include additional active agents against psoriasis. Pharmaceutical compositions according to the present invention can, therefore, be used both as monotherapy and administered to a patient in need of appropriate therapy in combination with one or more other therapeutic agents.

The features, advantages and characteristics of the CDK4/6 inhibitor apply likewise to the pharmaceutical composition according to the invention. Accordingly, in an embodiment of the pharmaceutical composition is provided as a skin-permeable formulation. Preferably the skin-permeable formulation is selected from the group consisting of: crème, gel, lotion.

Another subject-matter of the the disclosure relates to a method for the preparation of a pharmaceutical preparation comprising the formulation of the CDK4/6 inhibitor abemaciclib into a pharmaceutically acceptable carrier, wherein the CDK4/6 inhibitor is formulated into a skin-permeable formulation, preferably the skin-permeable formulation is selected from the group consisting of: crème, gel, lotion.

The features, advantages and characteristics of the CDK4/6 inhibitor apply likewise to the method according to the invention.

Another subject-matter of the disclosure relates to a method for the therapeutic treatment of a living being against psoriasis comprising the administration of the CDK4/6 inhibitor of the invention and/or the pharmaceutical composition of the invention to the living being, such as a human being.

The features, advantages and characteristics of the CDK4/6 inhibitor apply likewise to the method according to the disclosure.

Another subject-matter of the present disclosure relates to a method for the screening of active agents against psoriasis comprising the identification of a test compound's activity of a CDK4/6 inhibitor.

The findings of the inventors of the suitability of CDK4/6 inhibitors in the treatment of psoriasis allow the identification of novel active agents due to their CDK4/6-inhibitory activity. Assays to evaluate a test component's capability to inhibit the activity of CDK4/6 are well known to the skilled person, e.g. in form of competitive phosphorylation assays.

The features, advantages and characteristics of the CDK4/6 inhibitor apply likewise to the method according to the disclosure.

A still further subject-matter of the present disclosure relates to the use *ex vivo* of a CDK4/6 inhibitor for the suppression of the cellular IκBζ expression.

The inventors herewith provide a research tool not only allowing a better understanding of the transcriptional events up- and downstream of IκBζ expression but also the molecular mechanisms of psoriasis. According to the disclosure the use of the CDK4/6 inhibitor *in vitro* includes the addition of the active to a cell culture, e.g. a keratinocytes cell culture, and the evaluation of the molecular effects, resulting e.g. in altered gene expression, cytokine induction etc.

The features, advantages and characteristics of the CDK4/6 inhibitor apply likewise to the use according to the disclosure.

The features explained in the following do not only apply to the respective combination indicated or the specific embodiment but also in isolated position or to other combinations or to the invention in general without leaving the scope of the invention, which is defined in the claims. The embodiments serve to illustrate the invention but are not intended to restrict the scope of the invention. Reference is made to the enclosed figures which show:

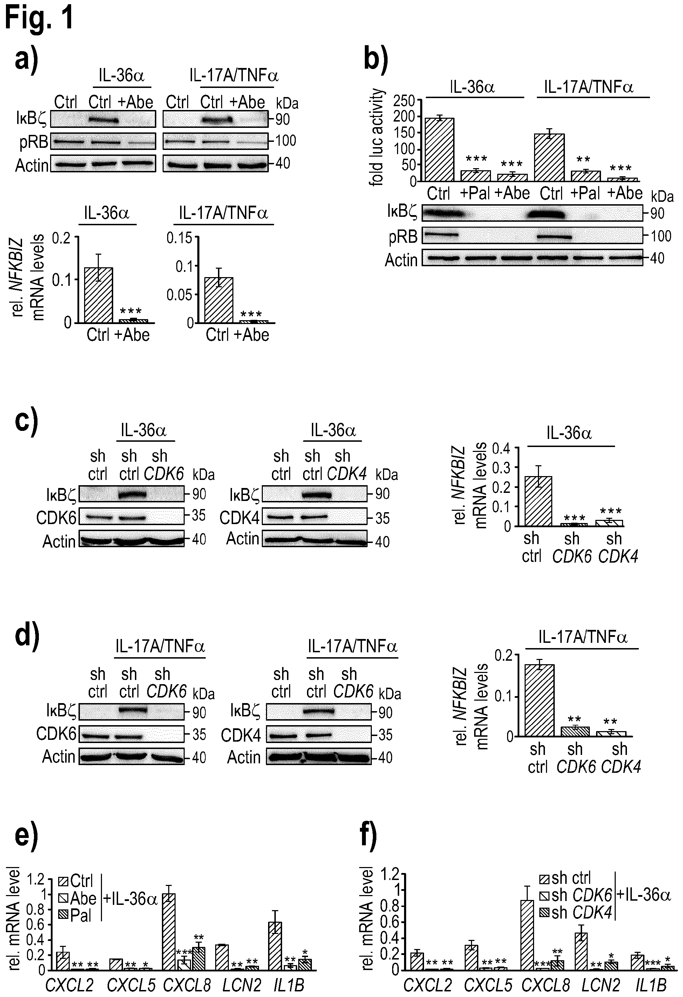

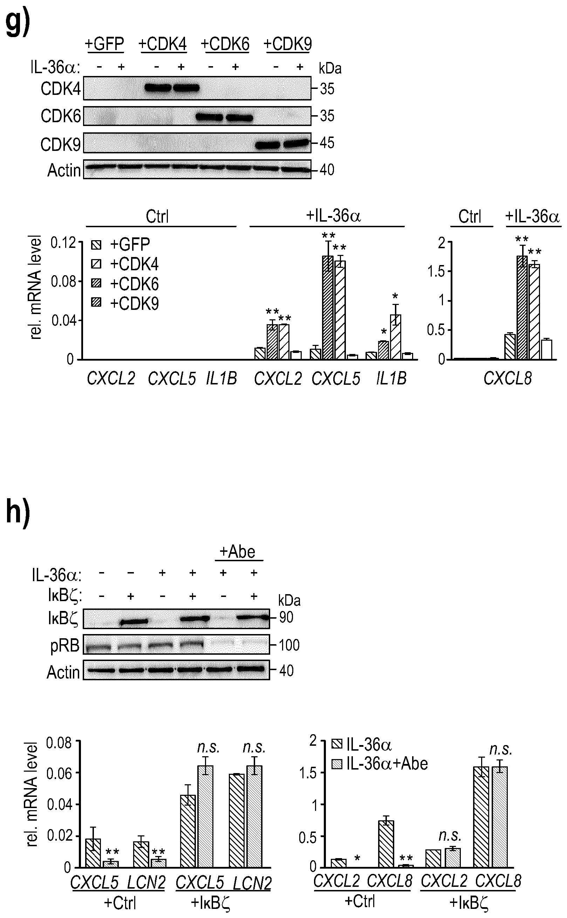

- Fig. 1:: CDK4/6 regulate the expression of IκBζ and its pro-inflammatory target genes in IL-36α- and IL-17A/TNFα-stimulated keratinocytes. a, Human primary keratinocytes were treated for 1 h with 100 ng/mL IL-36α or 200 ng/mL IL-17A and 10 ng/mL TNFα. The CDK4/6 inhibitor abemaciclib (Abe) or an ethanol vehicle control (Ctrl) were added in parallel. Phosphorylation of RB (pRB) served as a control for CDK4/6 inhibition, and Actin as a loading control. Relative mRNA levels of IκBζ (*NFKBIZ*) were normalized to the reference gene *RPL37A.* b, Luciferase assay of IκBζ (*NFKBIZ*) promoter activity in HaCaT cells that were cytokine-stimulated for 24 h in the presence or absence of the CDK4/6 inhibitors abemaciclib (Abe) or palbociclib (Pal). Relative luciferase (luc) activity was normalized to an internal Renilla luciferase control that was transfected in parallel. Endogenous protein levels were analyzed as input controls by immunoblotting (bottom). c, d, CDK4 and CDK6 were depleted in primary human keratinocytes by lentiviral transduction of shRNA. Ctrl shRNA- or *CDK4*/*6* shRNA-depleted cells were treated with (c) IL-36α or (d) IL-17A/TNFα, similar as in (a). e, f, Human primary keratinocytes were stimulated with IL-36α as in (a). e, Cytokine gene expression in CDK4/6 inhibitor-treated cells. f, Relative gene expression levels in IL-36α-treated control or *CDK4*/*6-*depleted cells. g, Transient overexpression of CDK4, CDK6 or CDK9 in HaCaT cells, treated for 1 h with 100 ng/mL IL-36α. h, Cytokine gene expression in HaCaT cells overexpressing IκBζ upon doxycycline pretreatment and stimulation with IL-36α in the presence or absence of abemaciclib. Significance: *p < 0.05; **p < 0.01; ***p < 0.001, *n.s.* = not significant.

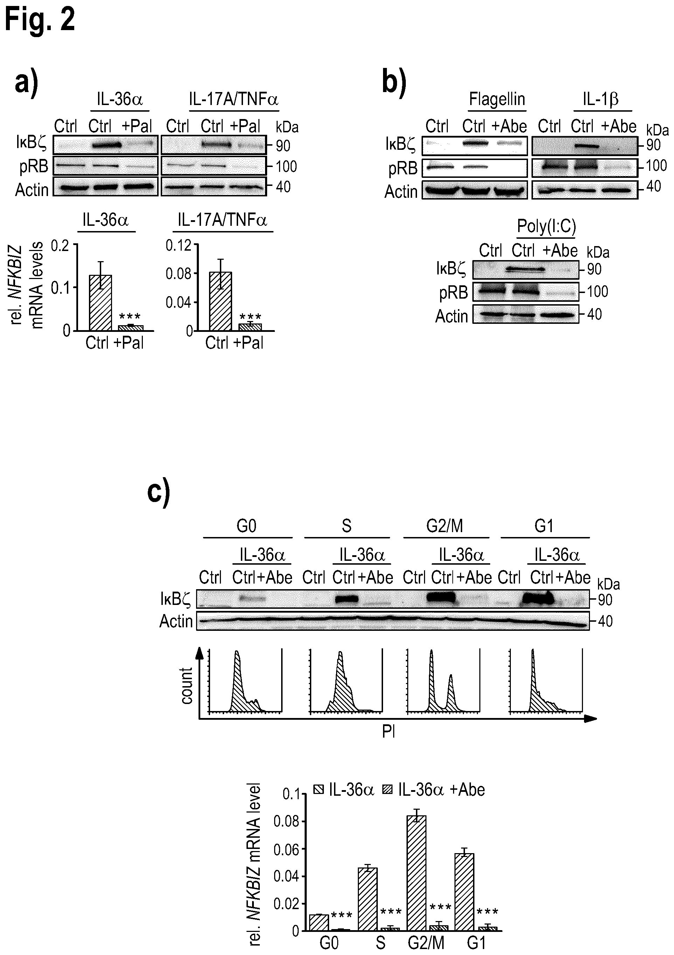

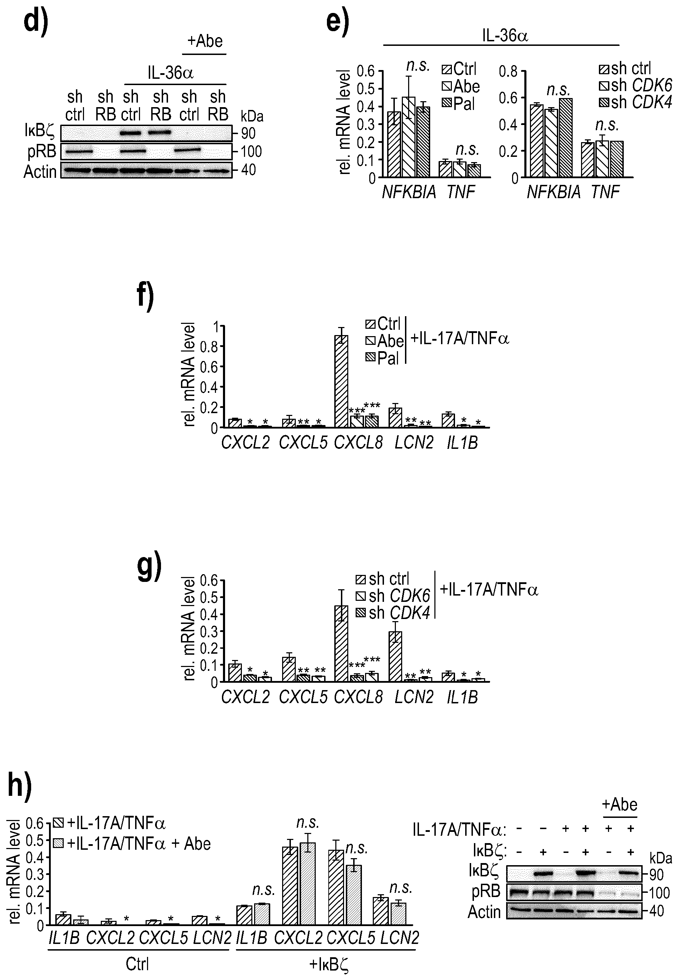

- Fig. 2:: Effects of CDK4/6 inhibition on IL-17A/TNFα-stimulated keratinocytes and analysis of possible cell cycle effects. a, IκBζ expression level in human primary keratinocytes stimulated 1 h with 100 ng/mL IL-36α or 200 ng/mL IL-17A and 10 ng/mL TNFα in presence or absence of palbociclib (Pal). Phosphorylation of RB (pRB) served as a control for CDK4/6 inhibition, Actin is the loading control. Relative mRNA levels of *NFKBIZ* were normalized to RPL37A. b, Keratinocytes were treated for 1 h with 100 ng/mL flagellin or 100 ng/mL IL-1β, or for 4 h with 10 ng/mL poly(I:C). Ethanol (Ctrl) or the CDK4/6 inhibitor abemaciclib (Abe) were added in parallel. Protein levels were analyzed as in (a). c, IκBζ expression in synchronized HaCaT cells. Cells were synchronized by double thymidine block. After release (0 - 16 h), cells were stimulated in the different cell phases for 1 h with 100 ng/mL IL-36α, in presence or absence of abemaciclib (Abe). The different cell cycle phases at the time of harvesting were controlled by PI staining. d, IκBζ protein level in RB-deficient HaCaT cells. Cells were treated with IL-36α and abemaciclib as in (c). e, Gene expression of IκBζ-independent genes in CDK4/6 inhibitor treated and CDK4/6-depleted keratinocytes. Stimulation as in (a). f + g, Gene expression of IκBζ target genes in human primary keratinocytes, stimulated for 1 h with 100 ng/mL IL-17A and 10 ng/mL TNFα. f, Gene expression in CDK4/6 inhibitor-treated cells. Concentration of the inhibitors as in (a). g, Gene expression in CDK4/6-deficient cells. h, Overexpression of IκBζ in HaCaT cells. Doxycycline-inducibly, IκBζ-expressing HaCaT cells were stimulated for 24 h with 2 µg/mL Doxycycline, followed by treatment with IL-17A/TNFα and abemaciclib as in (f). Significance: *p < 0.05; **p < 0.01; ***p < 0.001, n.s. = not significant.

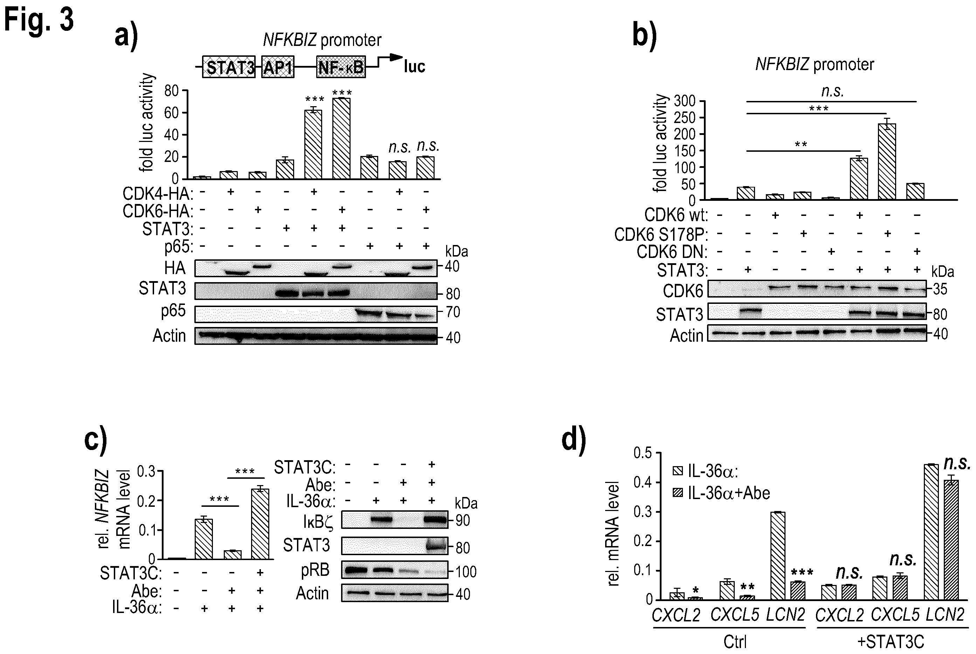

- Fig. 3:: STAT3 mediates CDK4/6-dependent IκBζ induction in keratinocytes. a, Luciferase assay of the *NFKBIZ* promoter in HEK293T cells after transient expression of CDK4, CDK6, STAT3 or p65, alone or in combination. The plasmid amounts for STAT3 (200 ng) and p65 (70 ng) were adjusted to achieve similar luciferase activity in the absence of CDK4/6 expression. Overexpression of the HA-tagged CDK4 and CDK6 proteins was detected using a HA-antibody. b, Luciferase activity assay of the *NFKBIZ* promoter in HEK293T cells overexpressing STAT3 alone or in combination with wildtype CDK6 (wt), hyperactive CDK6 (S178P) or a kinase-dead CDK6 mutant (CDK6 DN). c, HaCaT cells with a transient overexpression of hyperactive STAT3 (STAT3C) were treated for 1 h with 100 ng/mL IL-36α and abemaciclib (Abe). Left: *NFKBIZ* mRNA levels normalized to *RPL37A.* Right: Immunoblot analysis of STAT3C overexpression and CDK4/6 inhibition. d, IκBζ target gene expression in STAT3C-overexpressing HaCaT cells. Treatment as in (c). Significance: *p < 0.05; **p < 0.01; ***p < 0.001, *n.s.* = not significant.

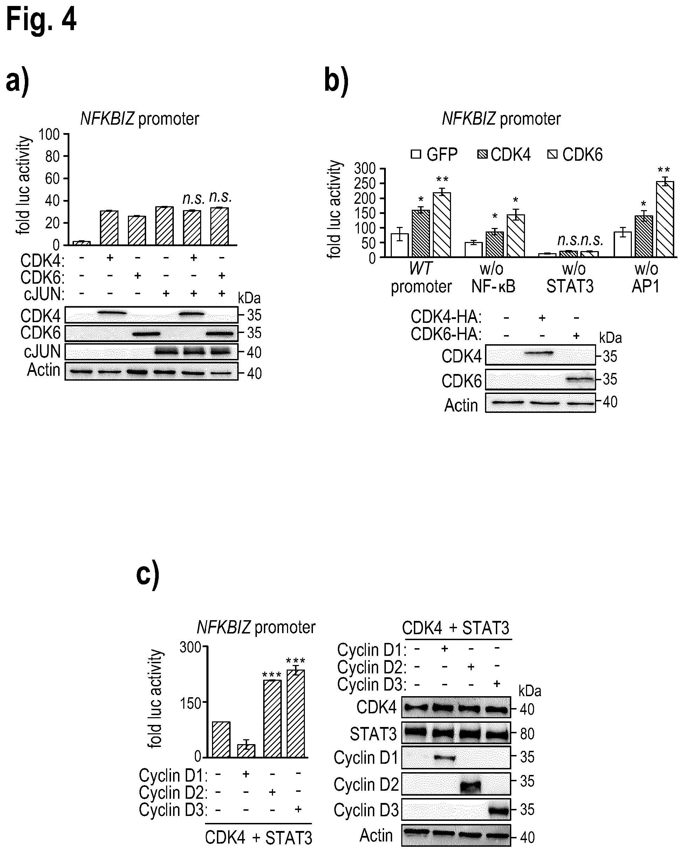

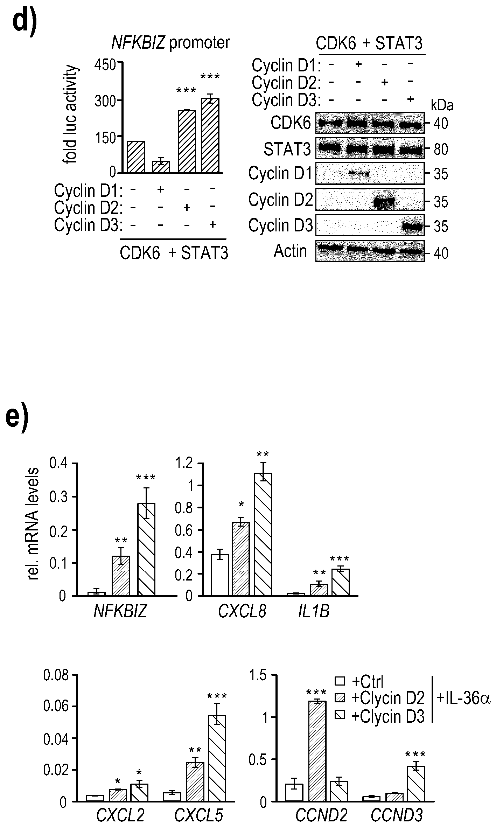

- Fig. 4:: CDK4/6 regulate STAT3-mediated IκBζ induction in a kinase-dependent manner. a, Analysis of the *NFKBIZ* promoter in HEK293T cells transiently overexpressing CDK4, CDK6 or cJUN, alone or in combination. Relative luciferase activity was normalized to co-transfected Renilla. b, Analysis of the *NFKBIZ* promoter in IL-36α-stimulated HaCaT cells using luciferase constructs that harbor deletions of NF-κB, STAT3 or AP1 binding sites. CDK4, CDK6 or GFP as control were transiently co-overexpressed in parallel. Relative luciferase activity was normalized to co-transfected Renilla. c + d, Luciferase assay of the *NFKBIZ* promoter in HEK293T cells, transiently overexpressing STAT3 and CDK4 (c) or STAT3 and CDK6 (d), alone or in combination with cyclin D1, cyclin D2 and cyclin D3 overexpression. e, Gene expression in HaCaT cells transiently overexpressing cyclin D2 or cyclin D3. Cells were stimulated for 1 h with 100 ng/mL IL-36α. Relative mRNA levels were normalized to RPL37A. (Ctrl = cells overexpressing empty control vector). Significance: *p < 0.05; **p < 0.01; ***p < 0.001, n.s. = not significant.

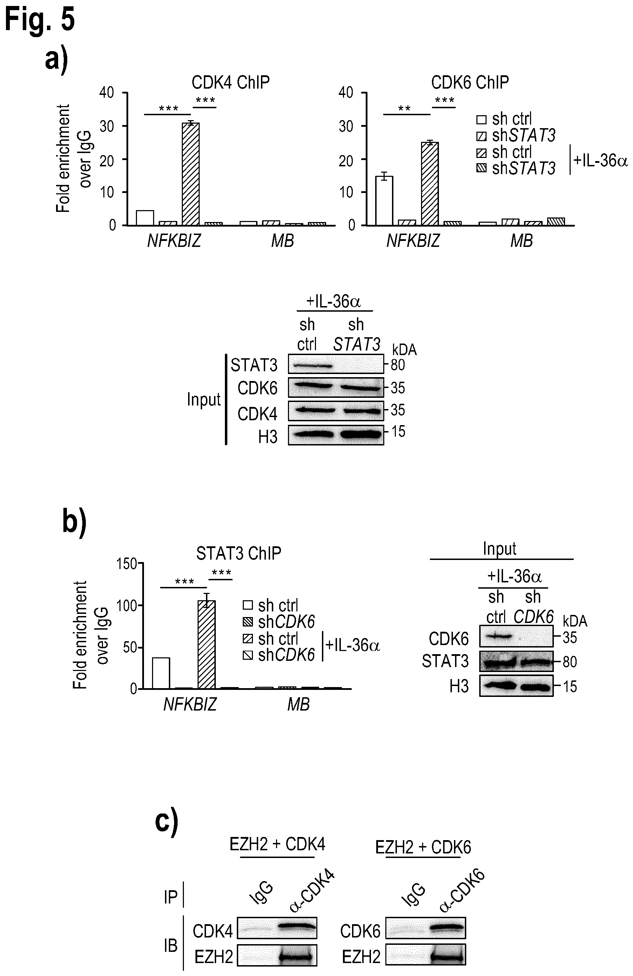

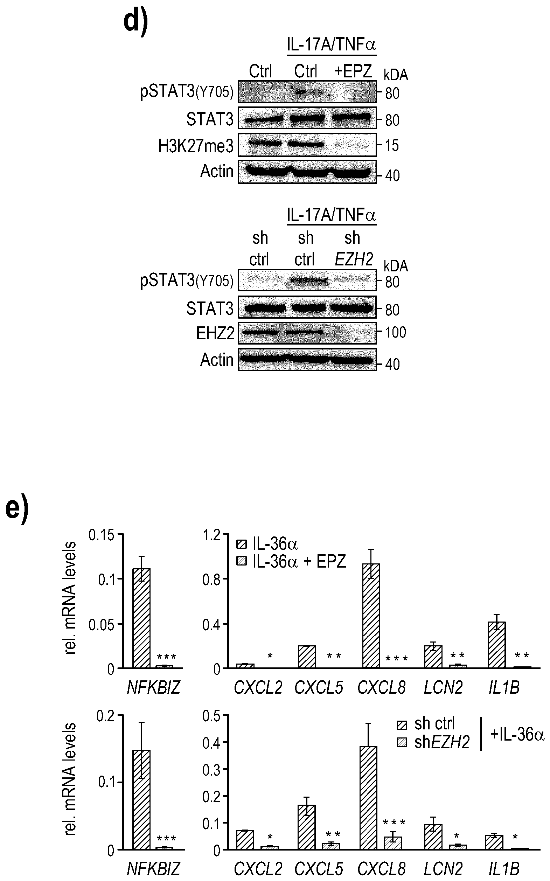

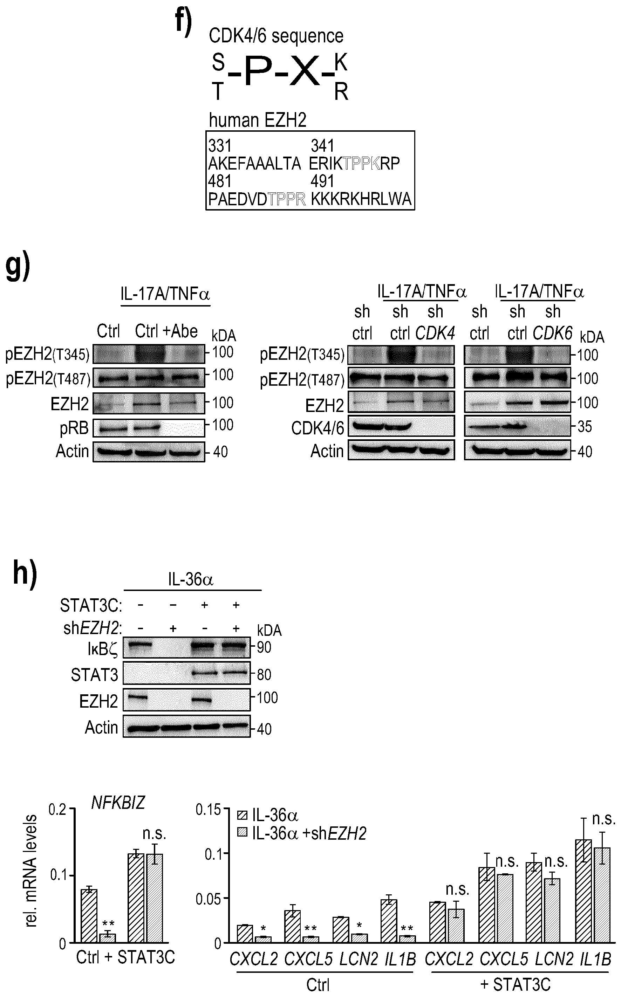

- Fig. 5:: Extended analysis of CDK4/6-mediated phosphorylation of EZH2 at T345 that induces STAT3 activation. a, Chromatin immunoprecipitation (ChIP) of CDK4, CDK6 or IgG as control. Control or STAT3-deficient HaCaT cells were treated for 30 min with 100 ng/mL IL-36α. Relative binding was calculated as the fold enrichment over IgG. (MB = Myoglobulin promoter; internal negative control). Equal CDK4/6 and STAT3 levels were controlled by immunoblot analysis of the ChIP input. b, STAT3-ChIP in IL-36α-stimulated, CDK6-deficient cells. Stimulation and analysis as in (a). c, Detection of CDK4/6 interaction with EZH2 in HEK293T cells. EZH2 was transiently overexpressed together with CDK4 or CDK6. CDK4/6-EZH2 complexes were pulled down using a CDK4- or a CDK6-specific antibody or IgG as control. d, STAT3 activity was analyzed by immunoblot detection of phosphorylated STAT3 (Y705) in human primary keratinocytes, treated for 1 h with 100 ng/mL IL-17A and 10 ng/mL TNFα in the presence or absence of an EZH2 inhibitor (EPZ6438, EPZ) or knockdown of EZH2. Detection of H3K27me3 controls effective EZH2 inhibition or depletion. e, Gene expression in EPZ6438-treated or EZH2-depleting, human primary keratinocytes. Cells were treated with 100 ng/mL IL-36α. mRNA levels were normalized to RPL37A. f, CDK4/6 substrate sequence and putative CDK phosphorylation sites of human EZH2 (marked in red). e, Immunoblot analysis of EZH2 activation by detection of phosphorylated EZH2 (T345) and (T487) in IL-17A/ TNFα-treated keratinocytes. Cells were treated as in (d), in the presence or absence of abemaciclib (Abe) or CDK4/6 knockdown. h, Analysis of IL-36α-mediated IκBζ induction and target gene expression in EZH2-depleted HaCaT cells, which overexpress a hyperactive STAT3 (STAT3C) version. Cells were treated for 1 h with 100 ng/mL IL-36α. Significance: *p < 0.05; **p < 0.01; ***p < 0.001.

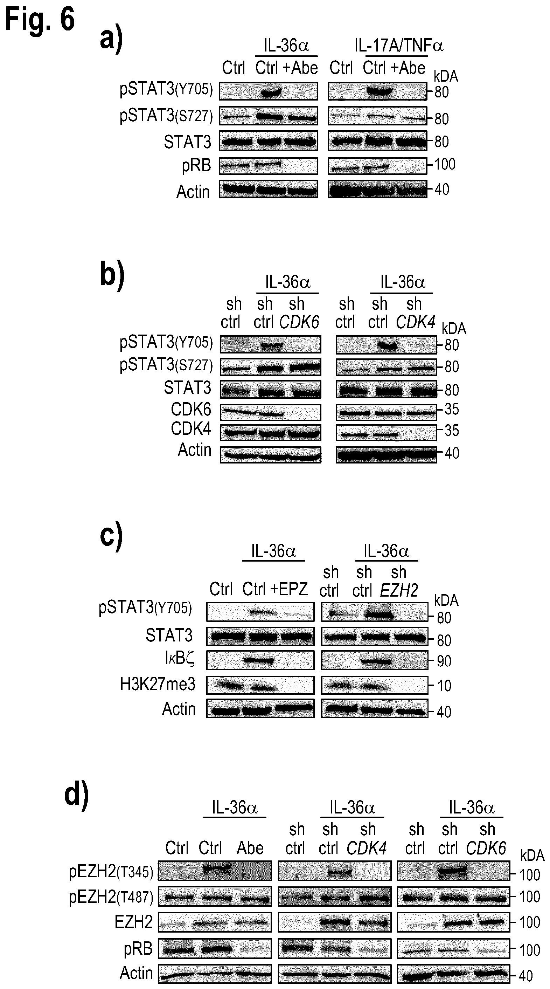

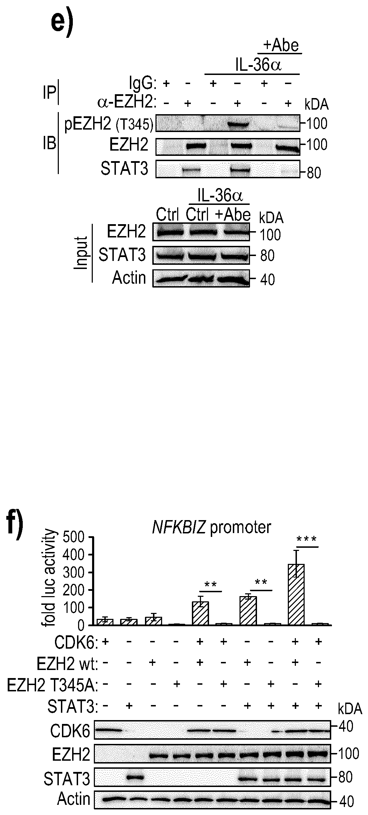

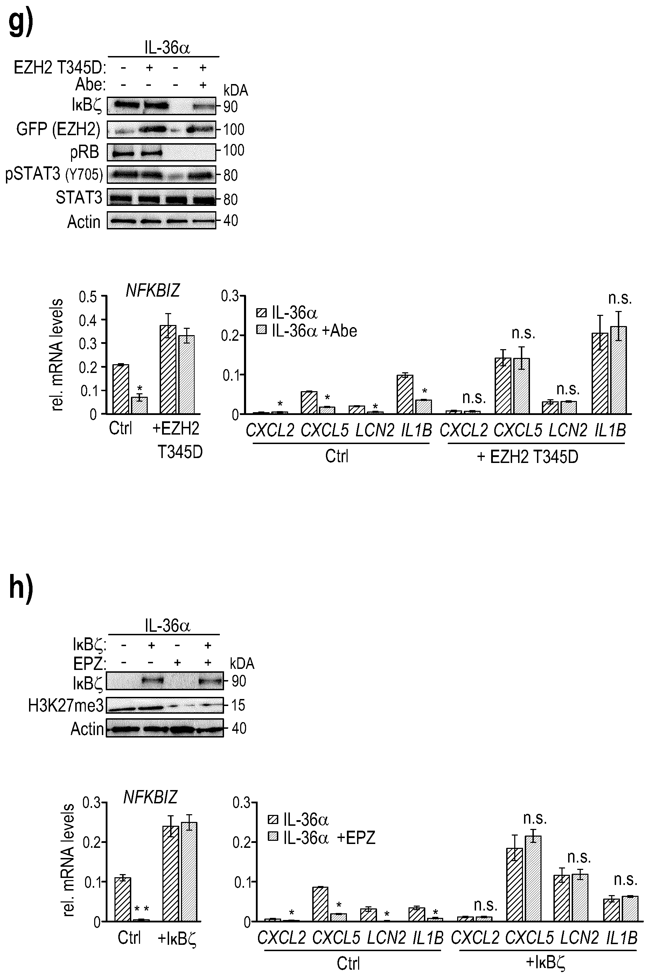

- Fig. 6:: CDK4/6 phosphorylate EZH2 to induce STAT3 activation. a, STAT3 activity was detected by analyzing the phosphorylation state at tyrosine 705 (Y705) and threonine 727 (T727) of STAT3 in primary human keratinocytes. After 2 h of starvation, cells were stimulated for 1 h with IL-36α or IL-17A and TNFα in the presence or absence of abemaciclib (Abe). b, STAT3 activity in *CDK4-* and CDK6-depleted keratinocytes. Stimulation as in (a). c, Immunoblot detection of phosphorylated STAT3 (Y705) in IL-36α-stimulated keratinocytes, in which EZH2 function was suppressed by the EZH2 inhibitor EPZ6438 (EPZ, 10 µM) or shRNA-mediated knockdown. Detection of H3K27me3 was used as a control for effective EZH2 inhibition or depletion. d, Immunoblot detection of phosphorylated EZH2 at threonine 345 (T345) and threonine 487 (T487) in abemaciclib-treated or *CDK4*/*6*-depleted keratinocytes following stimulation with IL-36α. e, Co-immunoprecipitation of EZH2 and STAT3 in HaCaT cells treated for 30 min with IL-36α in the presence or absence of abemaciclib. An EZH2-specific antibody or an unspecific IgG antibody as control were used for pulldown of protein complexes. STAT3 and pEZH2 (T345) were detected by immunoblotting. f, Luciferase activity assay of the *NFKBIZ* promoter in HEK293T cells, which transiently overexpress CDK6, wildtype EZH2 (wt), mutant EZH2 (T345A) or STAT3, alone or in combination. Equal protein expression was detected by immunoblotting (bottom). g, *NFKBIZ* and target gene expression in IL-36α- and abemaciclib-treated HaCaT cells following transient expression of a phospho-mimicking EZH2 (T345D) mutant. Left: Input controls. mRNA levels of *NFKBIZ* and its target genes were normalized to *RPL37A* (right). h, Doxycycline-induced ectopic expression of IκBζ overrides the inhibitory effects of the EZH2 inhibitor EPZ6438 (EPZ) on IL-36α-stimulated gene expression in in HaCaT cells. Significance: *p < 0.05; **p < 0.01; ***p < 0.001, *n.s.* = not significant.

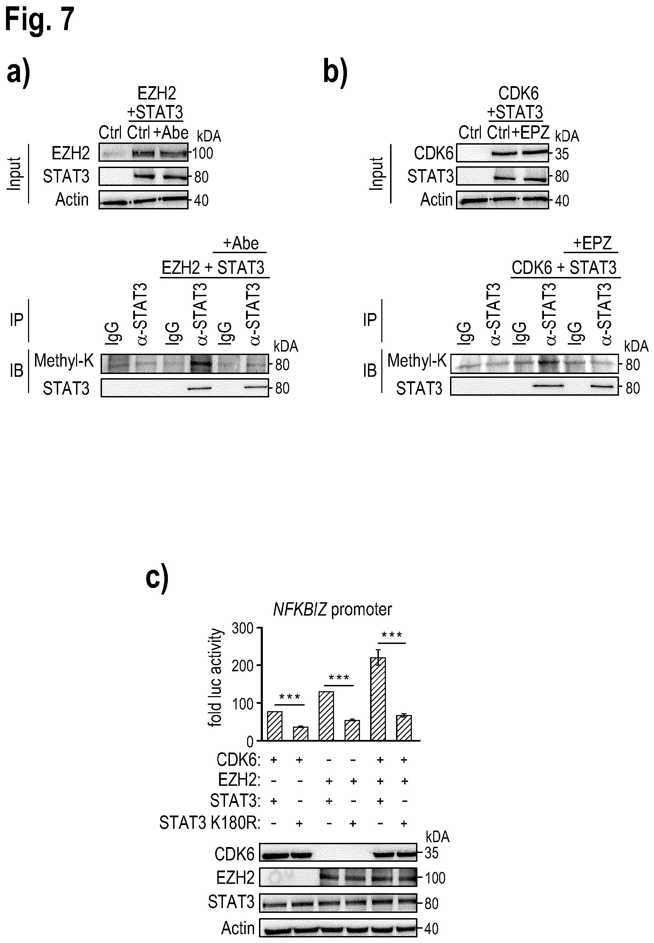

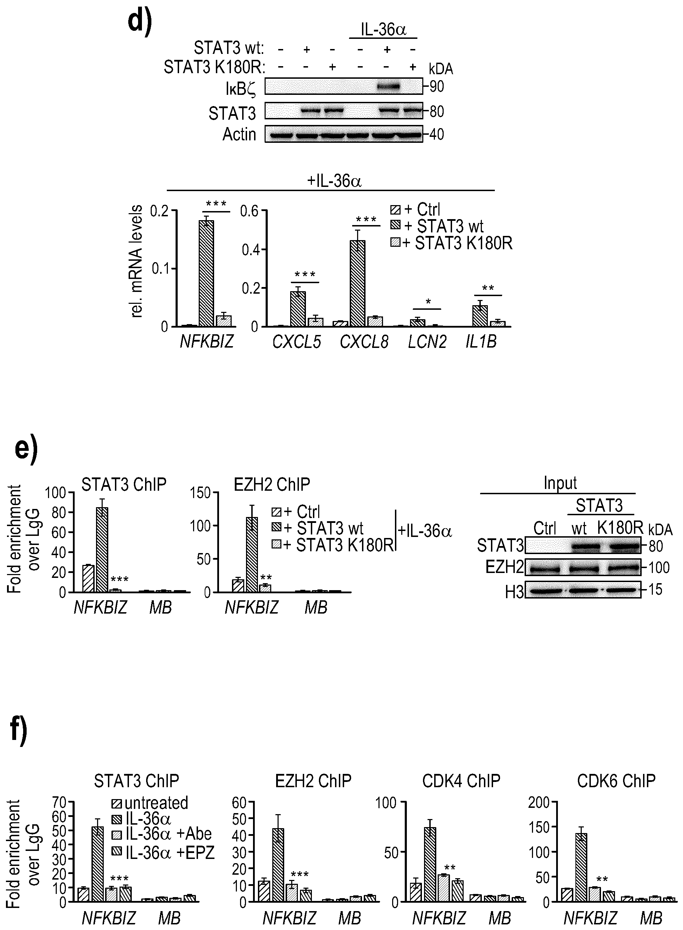

- Fig. 7:: CDK4/6-dependent, EZH2-mediated methylation of STAT3 at lysine 180 induces IκBζ expression in keratinocytes. a, b, Detection of methylated STAT3 by co-immunoprecipitation. EZH2 and STAT3 (a) or CDK6 and STAT3 (b) were transiently expressed in HEK293T cells. After 1 h of treatment with (a) abemaciclib (Abe) or (b) EPZ6438 (EPZ) cell lysates were prepared and subjected to immunoprecipitation using a STAT3-specific antibody or control IgG. *c, NFKBIZ* promoter-driven luciferase activity in HEK293T cells, transiently expressing CDK6 and EZH2, alone or in combination with wildtype (wt) STAT3 or methylation-defective STAT3 mutant (K180R). d, Analysis of IκBζ level and IκBζ target gene expression in STAT3 wt or STAT3 K180R expressing HaCaT cells. STAT3 wt or STAT3 K180R constructs were transiently expressed in *STAT3* KO HaCaT cells, followed by stimulation for 1 h with IL-36α. e, Chromatin immunoprecipitation (ChIP) of STAT3, EZH2 or IgG control in *STAT3* KO HaCaT cells reconstituted with either STAT3 wt or STAT3 K180R after 30 min of stimulation with IL-36α. Fold enrichment at the *NFKBIZ* promoter or at the myoglobin genomic region (*MB*; as negative control) was calculated rela- tive to the IgG control. f, ChIP of STAT3, EZH2, CDK4 and CDK6 in IL-36α-stimulated HaCaT cells stimulated for 30 min with IL-36α. Shown is the fold enrichment over IgG control. Significance: *p < 0.05; **p < 0.01; ***p < 0.001, *n.s.* = not significant. N=2.

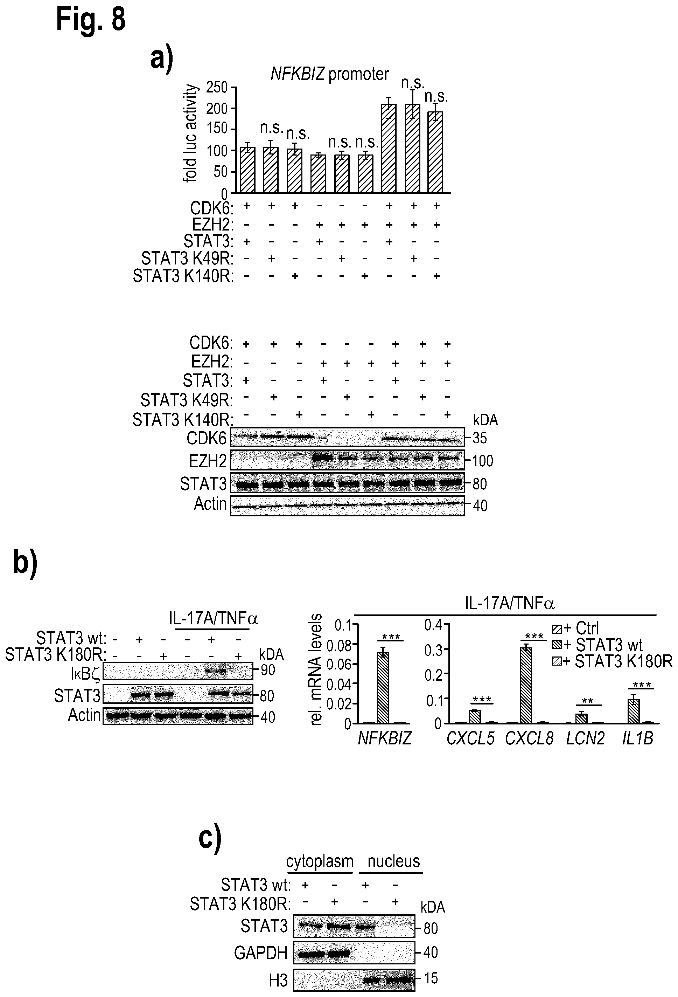

- Fig. 8:: Extended analysis of EZH2-mediated methylation of STAT3 in keratinocytes. a, Analysis of the *NFKBIZ* promoter in HEK293T cells transiently overexpressing CDK6 and EZH2, alone or in combination with wildtype STAT3 or mutant STAT3 K49R and K140R. Relative luciferase activity was normalized to co-transfected Renilla. b, Detection of IκBζ level and its target gene expression in STAT3 KO HaCaT cells, transiently overexpressing wildtype STAT3 or STAT3 K180R. Cells were treated for 1 h with 200 ng/mL IL-17A and 10 ng/mL TNFα. Relative mRNA levels of *NFKBIZ* and its target genes were normalized to RPL37A. c, STAT3 KO HaCaT cells, transiently overexpressing wildtype STAT3 or mutant STAT3 (K180R) were stimulated for 1 h with 100 ng/mL IL-36α, followed by nuclear fractionation of the cells and immunoblot analysis. GAPDH is a marker for the cytoplasmic fraction, H3 controls the nuclear fraction. Significance: *p < 0.05; **p < 0.01; ***p < 0.001.

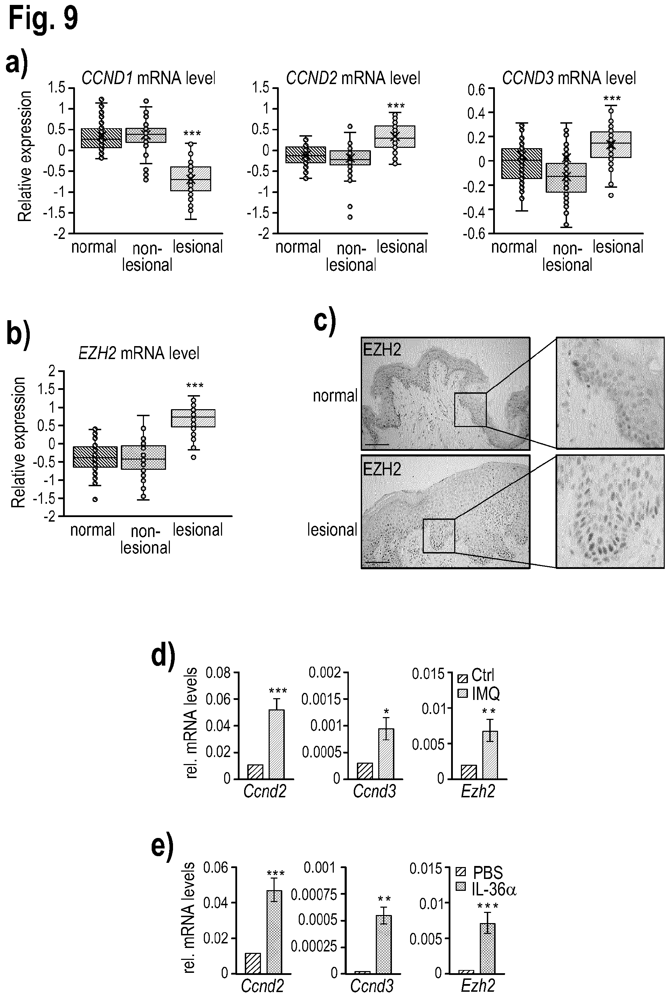

- Fig. 9:: Overexpression of cyclin D2, cyclin D3 and EZH2 in human and murine psoriasis. a, Expression data from skin biopsies of 64 healthy individuals and 58 psoriasis patients were analyzed from the GEO profile data set GDS4602. Shown are normalized expression values for *CCND1, CCND2* and *CCND3.* b, *EZH2* mRNA level in human skin samples from healthy and psoriasis patients; retrieved from the same data set as in (a). Significance: *p < 0.05; **p < 0.01; ***p < 0.001. c, IHC staining of EZH2 in normal and lesional skin. Scale bar: 100 µM. d, Analysis of *Ccnd2, Ccnd3* and *Ezh2* mRNA levels in IMQ-treated mice ears at day 7. Values were normalized to *Actin.* e, Analysis of *Ccnd2, Ccnd3* and *Ezh2* mRNA levels in IL-36α-treated mice ears at day 6. Significance: *p < 0.05; **p < 0.01; ***p < 0.001.

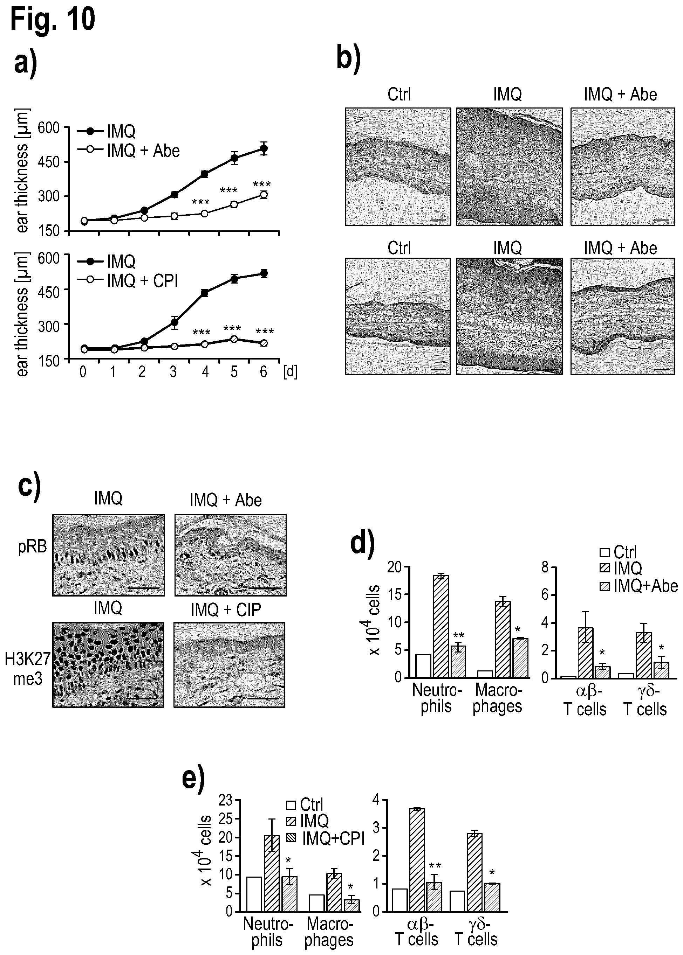

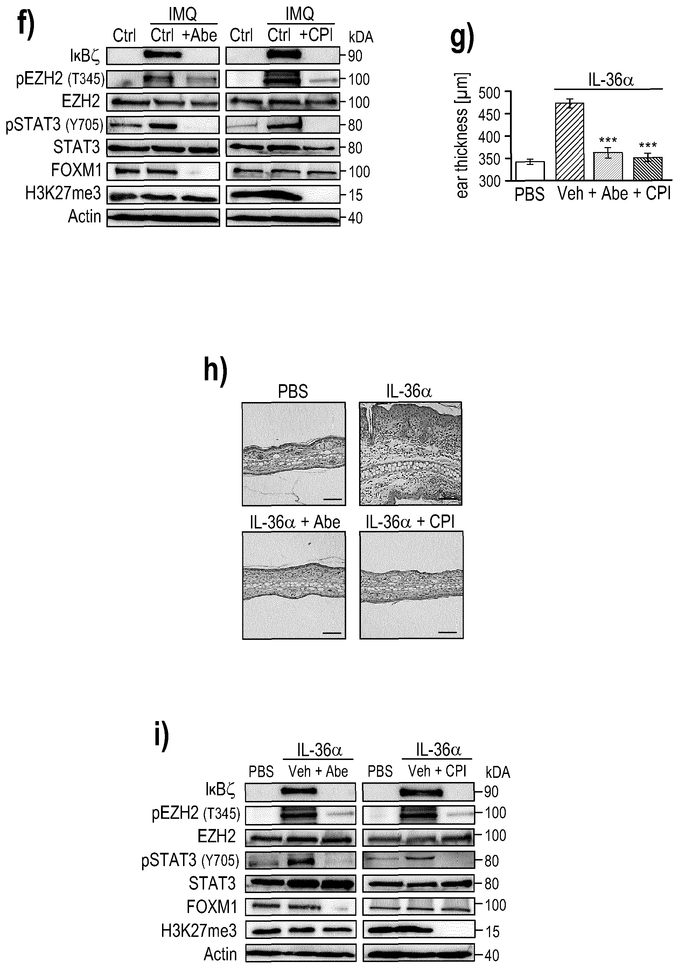

- Fig. 10:: CDK4/6 and EZH2 inhibition protects against imiquimod (IMQ)- and IL-36-mediated psoriasis *in vivo.* a, Ear thickness measurements during the topical treatment of mice with IMQ in the presence or absence of abemaciclib (Abe, 0.02 mg per ear/day; top) or the EZH2 inhibitor CPI-169 (CPI, 0.05 mg per ear/day; bottom). N = 6 mice per group +/- SEM. b, H&E staining of untreated (Ctrl), IMQ-, IMQ and Abe- or IMQ and CPI-treated ears. Scale bar: 100 µM. c, Phospho-RB (pRB) and H3K27me3 staining in ear skin sections of 6 day-treated mice controls effective CDK4/6 and EZH2 inhibition, respectively. Scale bar: 40 µM. d, Infiltrating immune cells in treated mouse ears at day 7 were quantified as the following: Neutrophils: CD45⁺, CD11b⁺, Ly6G⁺; Macrophages: CD45⁺, CD11b⁺, F4/80⁺; αβ-T cells: CD45⁺, CD3⁺, αβ-TCR⁺ and yδ-T cells: CD45⁺, CD3⁺, yδ-TCR⁺. N = 6 ears per group +/- SEM. e, Flow cytometry analysis of IMQ- or IMQ- and CPI-169-treated mouse ears at day 7. f, IκBζ expression, EZH2 phosphorylation (pEZH2 T345) and STAT3 activation (pSTAT3 Y705) were detected by immunoblot analysis of untreated (Ctrl) and IMQ-treated mouse skin tissue in the presence or absence of abemaciclib or CPI-169 at day 7. g, Ear thickness of IL-36α-treated mice at day 6. Ears of mice were daily treated by intradermal injections with 1 µg IL-36α. Control mice received injections with PBS. Additionally, mice received topical treatment with the ethanol vehicle control (Veh), 0.05 mg abemaciclib (Abe) or 0.02 mg CPI-169 (CPI). h, H&E staining of PBS- or IL-36α-treated ears at day 6. Scale bar: 100 µM. i, Immunoblot analysis of IκBζ, EZH2 phosphorylation (pEZH2 T345) and STAT3 activation (pSTAT3 Y705) in treated mouse skin tissue at day 6. FOXM1 and H3K27me3 were analyzed as positive controls for drug action. Significance: *p < 0.05; **p < 0.01; ***p < 0.001.

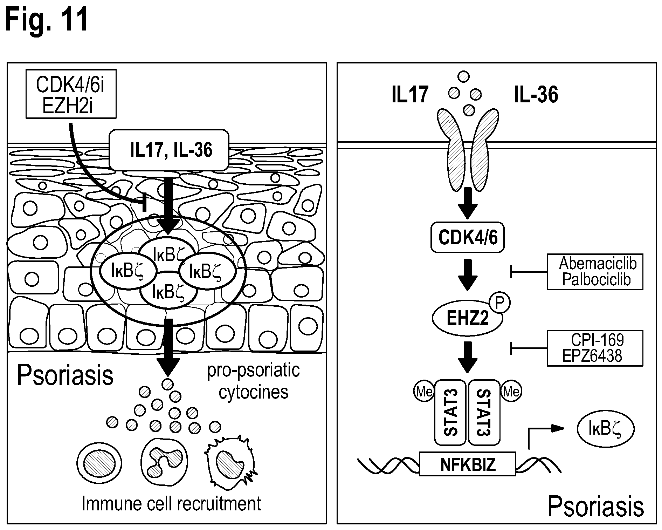

- Fig. 11:: The role of IκBζ and the CDK4/6-EZH2-STAT3 axis in keratinocytes and psoriasis. Left: IL-36 or IL-17 trigger the expression of IκBζ, which induces several target genes encoding for antimicrobial peptides and cytokines involved in the pathogenesis of psoriasis. Induction of IκBζ target gene expression results in the recruitment and activation of immune cells, thereby establishing psoriatic skin lesions. Topical application of CDK4 and EZH2 inhibitors on the skin blocks psoriasis pathogenesis. Right: Model of CDK4/6-EZH2-STAT3-mediated IκBζ expression in keratinocytes. Upon receptor binding of IL 17 and IL-36, CDK4/6 phosphorylates EZH2, thereby triggering a methylation- induced activation of STAT3, which is the main transcription factor involved in keratinocyte-derived IκBζ expression. Accordingly, inhibitors of both, CDK4/6 and EZH2, suppress cytokine-mediated IκBζ expression, thereby specifically abrogating the expression of psoriasis-related IκBζ dependent target genes.

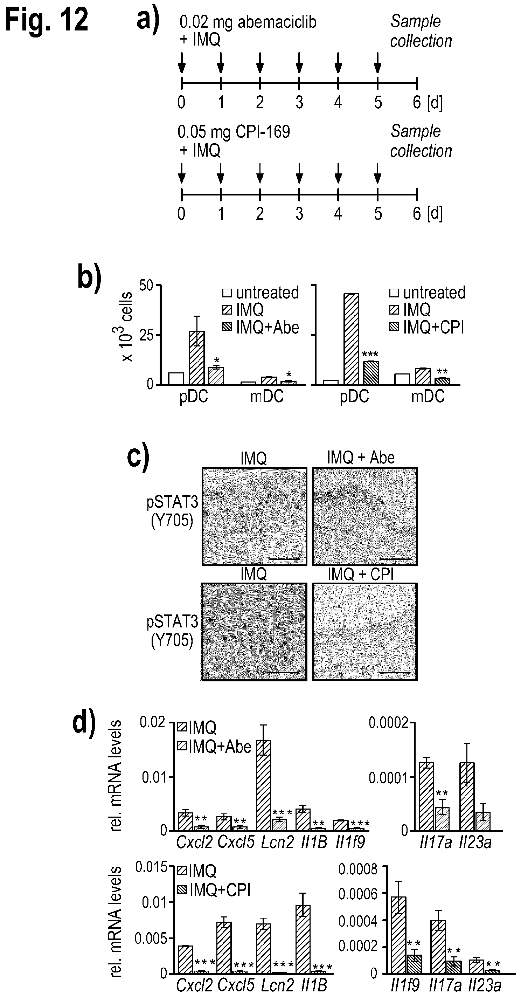

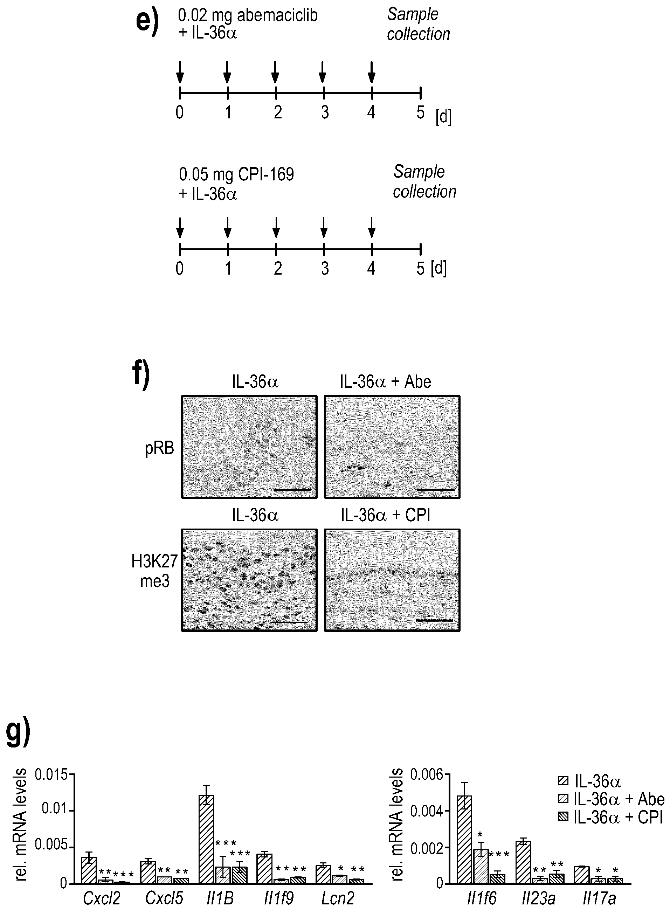

- Fig. 12:: Extended analysis of the effects of CDK4/6 or EZH2 inhibition on imiquimod (IMQ)- and IL-36-mediated psoriasis induction in vivo. a, Treatment scheme for induction of IMQ-mediated psoriasis in mice. Mice received daily application of IMQ-containing Aldara cream and topical application of abemaciclib (Abe, 0.02 mg/ per ear) or CPI-169 (CPI, 0.05 mg/ per ear). b, Characterization of infiltrating dendritic cell subsets into the ears of IMQ-treated mice by flow cytometry at day 7. Plasmocytoid dendritic cells (pDC) were detected as CD45+, CD11c+, MHC-II+, PDCA-1+, Siglec-H+, and myeloid derived dendritic cells (mDC) were analyzed as CD45+, CD11c+, MHC-II+, CD172a+. N = 6 ears per group +/- SEM. c, IHC staining of phosphorylated STAT3 at Y705 (pSTAT3) in the epidermis of treated mice at day 7. Scale bar: 40 µM. d, Gene expression analysis of IκBζ target genes in IMQ-, IMQ/Abe- and IMQ/CPI-treated skin samples at day 7. Relative mRNA expression was normalized to Actin. e, IL-36α treatment scheme with topical application of abemaciclib or CPI169 as in (c). 1 µg murine IL-36α or PBS control was intradermally injected into one ear of the mice for five consecutive days. f, IHC staining in ear skin sections of PBS or IL-36α-treated mice at day 6. pRB controls effective CDK4/6 inhibition and H3K27me3 serves as a marker for effective EZH2 inhibition. Scale bar: 40 µM. g, Gene expression of IκBζ target genes in IL-36α, IL-36α/Abe- and IL-36α/CPI-treated skin samples of 5 d treated mice. Significance: *p < 0.05; **p < 0.01; ***p < 0.001.

### Embodiments

### 1. Introduction

IκBζ represents an atypical member of the IκB family that is inducibly expressed in the nucleus, leading to the activation or repression of a selective subset of NF-κB target genes. Especially in keratinocytes, IL-17A, alone or even more potently in combination with TNFα, as well as IL-36 family cytokines, trigger a NF-κB- and STAT3-dependent transcriptional upregulation of IκBζ expression. Subsequently, IκBζ induces a subset of IL-36- and IL-17-responsive target genes in keratinocytes, including *CXCL2, CXCL5, CXCL8, LCN2, DEFB4* or *IL1B.* How IκBζ regulates these downstream target genes remains elusive though. It is assumed that IκBζ recruits epigenetic modifiers, such as TET2 or the SWI/SNF complex to the promoter sites of its target genes, leading to a change in DNA methylation or nucleosome remodeling.

IκBζ-deficient mice are completely protected against imiquimod (IMQ)- or IL-36-mediated psoriasis-like skin inflammation. Moreover, human psoriatic skin lesions are characterized by an upregulated expression of IκBζ; see Johansen et al. (2015; loc. cit.) and Müller et al. (2018; loc. cit.). As IκBζ lacks any enzymatic activity, it cannot be directly inhibited. Therefore, the inventors realized that small molecule inhibitors blocking the induction or downstream function of IκBζ could represent an alternative strategy for targeting IκBζ in psoriasis.

CDK4 and CDK6, in complex with cyclin D1, cyclin D2 or cyclin D3, represent well known cell-cycle regulating kinases that can phosphorylate RB, leading to the release of E2F transcription factors and G1-S cell cycle transition. Consistently, amplification of CDK4 and CDK6 as well as an overexpression of cyclin D proteins are frequently observed events in cancer, leading to the excessive proliferation of tumor cells. ATP-competitive CDK4/6 inhibitors, such as palbociclib and abemaciclib, have been developed for anticancer therapy and were recently approved for treatment of breast cancer patients. Interestingly, common side effects of a CDK4/6 inhibitor therapy constitute neutropenia and leukopenia. Moreover, it was found that CDK4/6 inhibition modulates immune cell functions in kinase-dependent or independent manners. Mechanistically, it is assumed that these atypical functions of CDK4 and CDK6 derive from their recently discovered role as cofactors for immune regulatory transcription factors. Especially chromatin-associated CDK6 can co-localize at promoter regions of a subset of NF-κB, STAT3 or AP1 target genes, thereby changing the DNA-binding properties or activity of these transcription factors.

In a screen for small-molecule inhibitors of IκBζ action in keratinocytes, the inventors identified CDK4/6 inhibitors as potent suppressors of IL-36- and IL-17A/TNFα-mediated IκBζ expression. Mechanistically, CDK4/6 inhibitors suppressed the activity of STAT3, which was identified as a major transcriptional regulator of IκBζ expression in keratinocytes. STAT3 activation was conveyed by CDK4/6-mediated phosphorylation of the methyltransferase EZH2, triggering the subsequent methylation of STAT3 and induction of IκBζ expression. Importantly, topical administration of CDK4/6 or EZH2 inhibitors on the skin completely prevented experimental psoriasis by suppressing STAT3 activation and consequently IκBζ expression in keratinocytes. Moreover, as cyclin D2, cyclin D3 and EZH2 were found to be overexpressed in human psoriatic skin lesions, the inventors propose repurposing of CDK4/6 inhibitors for topical skin treatment of psoriasis patients.

### 2. Methods

### Cell culture and treatment

HaCaT cells were maintained in DMEM with 10% FCS and antibiotics. Human primary keratinocytes were freshly isolated from foreskin and maintained in CnT-07S medium with gentamycin (CELLnTEC). Recombinant human IL-36α (aa 6-158) and mouse IL-36α (aa 6-160) were purchased from R&D Systems. Recombinant IL-17A (11340174), TNFα (11343013) and IL-1β (11340013) were ordered from Immunotools. Flagellin (vac-fla) and poly I:C (vac-pic) were purchased from Invivogen. In cell culture experiments, the cytokines were used at the following concentrations, as described previously: IL-36α (100 ng/mL), IL-17A (200 ng/mL), TNFα (10 ng/mL) and IL-1β (100 ng/mL). Flagellin was applied at 10 ng/mL and poly (I:C) was added at a final concentration of 100 ng/mL. The following inhibitors were purchased from Selleckchem: Abemaciclib mesylate (LY2835219, S17158), Palbociclib isethionate (S1579), EPZ6438 (tazemetostat, S7128) and CPI-169 (S7616). If not otherwise indicated, the inhibitors were used in cell cultures at the following concentrations: abemaciclib (16 µM), palbociclib (50 µM) and EPZ6438 (10 µM). When indicated, cells were starved for 2 h prior to cytokine treatment, by removing cell culture supplements from the growth medium.

### Generation of knockdown cells

Lentiviral particles were produced in HEK293T cells using the vector pMD2.G and a second-generation packaging system (psPAX2, Addgene). Keratinocytes were transduced in the presence of 8 µg/mL polybrene, packaging plasmids and 5 µg of the respective shRNA construct: pLKO.1-puro (sh ctrl); pLKO.1-TRCN0000009876 *(shCDK4);* pLKO.1-TRCN0000010473 (shCDK6); pTRIPZ-EZH2 (V2THS63066, Dharmacon); pGIPZ noncoding ctrl (Dharmacon, RHS4351); pLKO.1-TRCN0000020840 (shSTAT3, Sigma); pLKO.1-TRCN0000014683 (shRELA, Sigma), followed by puromycin selection (1 ng/mL, Invitrogen).

### Luciferase constructs and reporter assays

Luciferase constructs were generated as described; Muller et al. (2018; loc. Cit.). 1*10⁴ HEK293T cells were transfected for 24 h using HBS buffer and CaCl₂ and a mixture of 400 ng luciferase vector and 100 ng TK-Renilla vector. For expression of other factors, the following concentrations were used: 70 ng p65, 200 ng cJUN, STAT3 or EZH2 constructs and 500 ng CDK4, CDK6 or cyclin D expression constructs (not shown). For transfection of HaCaT cells, 3*10⁵ cells were transfected for 4 h using Lipofectamine 3000 and a mixture of 800 ng luciferase vector, 200 ng TK-Renilla vector and 4 µg expression or control plasmids according to the manufacturer's instructions (Thermo Fisher). 36 h after transfection, luciferase activity was measured with the Dual Luciferase Reporter Assay Kit (Promega). Expression of the reporter constructs was calculated as the fold induction over unstimulated transfected cells from data of three independent experiments.

### Transient overexpression in HEK293T and HaCaT cells

HEK293T cells were transfected using HBS buffer and CaCl₂. HaCaT cells were transfected with Lipofectamine 3000, according to the manufacturer's instructions (Thermo Fisher). 5 µg expression constructs were incubated with 3*10⁵ cells for 4 h. 36-48 h post transfection, cells were harvested and analysed.

### Doxycycline-inducible IκBζ overexpression

IκBζ was cloned into the lentiviral plnducer20 plasmid (Addgene, 44012) using pENTR TOPO cloning. After lentivirus production in HEK293T cells, HaCaT cells were transduced and selected with 450 µg/mL G418 (Invivogen). Induction of IκBζ expression cells was achieved by doxycycline treatment (2 µg/ml, AppliChem) for 24 h.

### Generation of STAT3 mutants

Mutation of STAT3 at K49, K140 and K180 was performed by site-directed mutagenesis of the human STAT3 pcDNA3 construct from Addgene (71447), which was previously cloned into the Strep-tagged backbone (pEXPR-IBA103). Substitution of the amino acid was performed with self-designed primers (not shwon).

### CRISPR/Cas9 gene editing of STAT3 KO HaCaT cells

The CRISPR/Cas9 one vector system was used to generated STAT3 KO HaCaT cells according to well-known methods. The guide RNA against STAT3 (forward: 5'-CACCGACTGCTGGTCAATCTCTCCC-3⁻ (SEQ ID NO:1), reverse: 5'-AAACGGGAGAGATTGACCAGCAGTC-3⁻ (SEQ ID NO:2)) was cloned into the len-tiCRISPRv2 containing Cas9 vector (Addgene, 52961), followed by lentiviral transduction and puromycin selection.

### Synchronization of HaCaT cells

Synchronization of the cells with a double thymidine block was performed as described before. After the second thymidine block, cells were released in normal medium. At 0, 4, 10 and 14 h after release, cells were stimulated with IL-36α and/or abemaciclib for 1 h. Propidium iodide staining was performed by flow cytometry (LSRII, Becton Dickson) to detect the cell cycle phase at the time point of cell harvest.

### Western blot analysis

Western blot analysis was performed as described before. The following antibodies were used and purchased from Cell Signaling: anti-IκBζ (9244), anti-phospho-STAT3 at Tyr705 (9145), anti-phospho-STAT3 at Ser727 (9134), anti-STAT3 (12640), anti-p65 (8242), anti-EZH2 (5246), anti-pRB (phospho-RB at Ser807/811; 8516), anti-FoxM1 (5436), anti-H3 (4499), anti-CDK4 (12790), anti-CDK6 (13331), anti-CDK9 (2316), anti-cyclin D1 (2978), anti-cyclin D2 (3741), anti-cyclin D3 (2936), anti-cJUN (9165), anti-H3K27me3 (9733), anti-GAPDH (2118), anti-H3 (9715) and anti-β-actin (3700). Anti-β-Gal (sc377257) and anti-GFP (sc9996) were obtained from Santa Cruz Biotechnology. Anti-pEZH2 at T345 (61242) anti-pEZH2 at T487 (12820) were purchased from Active Motif and anti-pan-methyl-lysine antibody was purchased from Enzo (ADI-KAP-TF121-E). For detection of mouse IκBζ, a self-made antibody raised against peptides CSAPGSPGSDSSDFSS (SEQ ID NO:3) and CLHIRSHKQKASGQ (SEQ ID NO:4) was applied.

### Chromatin immunoprecipitation (ChIP)

ChIP assays were performed as described before. After sonification, chromatin was incubated with protein G-coupled Dynabeads (Invitrogen) and 2 µg of STAT3 (Thermo Fisher, MA1-13042), CDK4 (Cell Signaling, 12790), CDK6 (Sigma, HPA002637), EZH2 (Diagenode, C15410039) or control IgG antibody (Abcam, ab46540) overnight at 4°C. The promoter region of myoglobulin (MB) served as an internal negative control [forward: 5'-CTCTGCTCCTTTGCCACAAC-3⁻ (SEQ ID NO:5), reverse: 5'-GAGTGCTCTTCGGGTTTCAG-3⁻ (SEQ ID NO:6)]. ChIP primers corresponding to the promoter region of *NFKBIZ* were self-designed [forward 5'-GCCTTAACTGGGCTAACAGC-3' (SEQ ID NO:7), reverse 5'-CTGGCAAGTCCTGGAAGGAG-3' (SEQ ID NO:8)]. Data from two independent experiments is presented as the fold enrichment, calculated over the percentage of input from the IgG control ChIP.

### Co-Immunoprecipitation (CoIP)

Cells were lysed by mechanical disruption using standard lysis buffer (50 mM Tris-HCl pH 7.5, 150 mM NaCl, 1% NP-40, 1x Protease inhibitor cocktail, Roche). Subsequently, lysates were sonicated for 5 minutes at high power (Bioruptor, Diagenode), followed by pre-clearing of the lysates with protein A/G PLUS agarose beads (Santa Cruz, sc-2003) for 1 h at 4°C. Precleared lysates were incubated either with antibodies specific for CDK4 (Cell Signaling, 12790), CDK6 (Sigma, HPA002637), EZH2 (Cell Signaling, 5246), STAT3 (MA1-13042, Thermo Fisher,) or β-Gal antibody (sc-19119, Santa Cruz) as an IgG control, overnight at 4°C. For endogenous IPs immune complexes were precipitated with protein A/G PLUS agarose beads and eluted by 6x SDS-PAGE sample buffer.

### Gene expression analysis by qPCR

Gene expression analyses were performed as described. Relative gene expression was analyzed using self-designed primers ordered at Metabion (not shown). Relative mRNA levels were calculated by normalization to the human reference gene *RPL37A* or the mouse reference gene *Actin* using the 2-ΔΔCt method.

### Mice

Experiments were conducted in accordance with the German law guidelines of animal care. Ears of female C57BL/6 mice (8-12 weeks old) were topically treated for 6 consecutive days with 5 mg Aldara cream (containing 5% imiquimod, 3M Pharmaceutical) and 0.02 mg of abemaciclib (in 10 µL ethanol), 0.05 mg CPI-169 (in 10 µL ethanol) or vehicle control. At day 7, mice were sacrificed and analysed. For IL-36α-mediated psoriasis induction, ears of male C57BL/6 mice (8-12 weeks old) were treated by intradermal injections using 1 µg murine IL-36α (aa 8-160, R&D) or PBS control for 5 consecutive days. For application of the vehicle control, abemaciclib (0.02 mg) or CPI-169 (0.05 mg), substances were mixed with Miglyol 812 (Carl Roth) in a ratio of 1:4. Inhibitors were topically applicated 6 h before intradermal injections of IL-36α or PBS were applied. Mice were sacrificed and analysed at day 6.

### Flow cytometry

Sample preparation was performed as described. The following anti-mouse antibodies from BioLegend were used: anti-CD45 FITC (103107), anti-CD11b PacificBlue (101223), anti-Ly6G PE (127607), anti-F4/80 APC (123115), anti-CD11c Pacific Blue (117322), anti-MHC-II APC (107613), anti-CD172a PE (144011) and anti-Silec-H PE (129605). Anti-PDCA-1 APC (17-2092-80) and anti-αβTCR PE (HM3628) were purchased from Invitrogen, and anti-γδ-TCR APC (17-5711-82) from Sigma. Data was acquired on a LSRII flow cytometer (Becton Dickson).

### Histology

Ear sections from mice were fixed in 10% formalin (Carl Roth) and subsequently embedded in paraffin. 5-µm sections were prepared and incubated with the following antibodies from Cell Signaling: pSTAT3 (9145), pRB (8516) and H3K27me3 (9733). For staining of human skin samples, EZH2 antibody (Cell Signaling, 5246) was used. Antigen retrieval was performed in 1 mM EDTA pH 8.0 for pSTAT3, and 10 mM citrate buffer pH 6.0 + 0.5% Triton X-100 for EZH2, H3K27me3 and pRB. After incubation with peroxidase-coupled secondary antibodies, sections were stained with DAB substrate.

### Analysis of patient data

Gene expression data originated from the GEO data set GSE13355; see Nair, R.P., *et al.,* Genome-wide scan reveals association of psoriasis with IL-23 and NF-kappaB pathways, Nat Genet 41, 199-204 (2009); and Swindell, W.R., et al., Genome-wide expression profiling of five mouse models identifies similarities and differences with human psoriasis. Plos One 6, e18266 (2011). Pre-normalized gene expression values from each sample was directly taken from the GEO profile data set GDS4602. The following reporters were taken for analysis: *EZH2:* ID 203358_s_at, *CCND1:* ID 208711_s_at, *CCND2:* ID 200953_s_at and *CCND3:* ID 201700_s_at.

### 3. Results

### CDK4/6 inhibitors suppress IκBζ expression and IκBζ-dependent pro-inflammatory gene expression in IL-36α- and IL-17A/TNFα-stimulated keratinocytes

Due to a lack of enzyme activity, direct inhibition of IκBζ is not feasible. Key regulators in psoriasis constitute IL-17 and IL-36 family members, which predominantly trigger a pro-inflammatory response in keratinocytes that is dependent on IκBζ. Thus, the inventors performed an unbiased screen for small-molecule inhibitors that are able to block induction of IκBζ expression in response to either IL-36α or IL-17A. Previously, it was shown that IL-17-induced IκBζ expression is strongly increased in combination with TNFα; see Johansen (2015; loc. cit.) and Müller (2018; loc. cit.). Intriguingly, the inventors found that two CDK4/6 inhibitors, abemaciclib (Fig. 1a) and palbociclib (Fig. 2a), completely blocked IL-36α- or IL-17A/TNFα-mediated induction of IκBζ expression in primary human keratinocytes. Moreover, the inventors observed similar effects in response to IL-1β or the TLR ligands flagellin and poly(I:C) (Fig. 2b), thereby revealing a conservation of this pathway in keratinocytes.

To explore whether these effects were due to a CDK4/6 inhibitor-mediated G1-cell cycle arrest, the inventors repeated the experiments in synchronized and single cell cycle phase-arrested keratinocytes. IL-36α treatment triggered IκBζ induction largely in all phases of the cell cycle, which was completely suppressed by abemaciclib (Fig. 2c). Importantly, although a minor, IL-36-mediated only a week induction of IκBζ expression in G₀ phase. Depletion of *RB* by RNA interference did not influence IL-36-mediated IκBζ induction (Fig. 2d), thereby clearly indicating that the effect of CDK4/6 inhibitors on IκBζ was independent of their ability to trigger cell cycle arrest. Instead, the inventors revealed that CDK4/6-dependent induction of IκBζ was mediated at the transcriptional level, as palbociclib and abemaciclib treatment abrogated the expression of a luciferase construct harboring the *NFKBIZ* (IκBζ) promoter in IL-36α-stimulated HaCaT cells (Fig. 1b). Interestingly, also shRNA-mediated depletion of *CDK4* or *CDK6* was sufficient to suppress IL-36α- or IL-17A/TNFα-dependent expression of IκBζ in human primary keratinocytes, thereby excluding any off-target effects of the applied inhibitors (Fig. 1c and 1d). Accordingly, IκBζ-dependent target genes, such as *CXCL2, CXCL5* or *CXCL8*, were strongly downregulated in IL-36α- and CDK4/6 inhibitor-treated keratinocytes (Fig. 1e), as well as in *CDK4-* or *CDK6*-deficient cells (Fig. 1f), whereas other NF-κB-dependent, but IκBζ-independent genes, such as *NFKBIA* or *TNF,* remained unaffected (Fig. 2e). Similar effects of pharmacological or shRNA-mediated inhibition of CDK4/6 were obtained in IL-17A and TNFα-stimulated cells (Fig. 2f and 2g). CDK4/6 inhibitors have the potential to inhibit CDK9 kinase activity, although much higher concentrations are needed. To rule out effects deriving from the suppression of CDK9 activity, the inventors transiently overexpressed CDK4, CDK6 or CDK9 in HaCaT cells and analyzed IL-36α-mediated gene expression. CDK4 and CDK6 overexpression, but not CDK9, could increase IL-36α-mediated, IκBζ-dependent target gene expression in keratinocytes, thereby further confirming the specificity of CDK4 and CDK6 in regulating pro-inflammatory target gene expression in keratinocytes (Fig. 1g).

The inventors hypothesized that CDK4/6 are not involved in the direct regulation of IκBζ target gene expression but rather trigger the expression of IκBζ, which in turn induces a secondary, IκBζ-dependent gene expression in stimulated keratinocytes. To test this hypothesis, the inventors overexpressed a doxycycline-inducible version of IκBζ in IL-36α- or IL17A/TNFα-stimulated HaCaT cells in the presence or absence of abemaciclib (Fig. 1h and Fig. 2h). In this set-up, exogenous overexpression of IκBζ completely abolished abemaciclib-mediated suppression of IκBζ target gene expression, thereby validating CDK4/6-mediated transcriptional upregulation of IκBζ as a pre-requisite for CDK4/6-dependent pro-inflammatory gene expression in keratinocytes.

### CDK4/6-dependent induction of IκBζ expression is mediated by STAT3

Beside their known involvement in cell cycle regulation, CDK4 and CDK6 have previously been described to function as transcriptional cofactors for STAT3, NF-κB or AP-1. As the inventors revealed a CDK4/6-dependent induction of IκBζ on the transcriptional level, the inventors next explored the responsible transcription factor. Of note, binding sites for all three transcription factors were previously identified at the *NFKBIZ* promoter region. Interestingly, expression of both CDK4 and CDK6 increased the STAT3-mediated induction of *NFKBIZ* promoter activity, whereas no synergistic effects could be observed when CDK4/6 were co-overexpressed with NF-κB p65 or cJUN (Fig. 3a and Fig. 4a). In agreement, deletion of the STAT3-binding site abrogated the expression of the *NFKBIZ* luciferase reporter in IL-36α-stimulated, CDK4/6-overexpressing HaCaT cells, whereas deletion of the NF-κB or AP1 motif had only a minor or almost no effect (Fig. 3b).

Previous publications reported that CDK6 acts as a cofactor for STAT3, independently of its kinase function. Therefore, the inventors tested if a kinase-dead mutant of CDK6 (CDK6 DN) could still synergize with STAT3 in driving the expression of the *NFKBIZ* luciferase reporter construct. Surprisingly, the kinase-dead mutant was not able to cooperate with STAT3 anymore, whereas a hyperactive version of CDK6 (CDK6 S178P) further increased the activity of the *NFKBIZ* promoter in a STAT3-dependent manner (Fig. 3b). Accordingly, cyclin D2 and cyclin D3, which associate with CDK4/6 to activate their kinase function, synergized with CDK4/6 and STAT3 in activating the *NFKBIZ* luciferase promoter, whereas cyclin D1 failed to do so (Fig. 4c and 4d). Moreover, cyclin D2 and cyclin D3 overexpression significantly elevated the expression of *NFKBIZ* and its target genes in IL-36α-stimulated keratinocytes (Fig. 4e). Of note, transient overexpression of a constitutively active STAT3 mutant (STAT3C) abrogated the inhibitory effects of CDK4/6 inhibition on the induction of IκBζ (Fig. 3c) and IκBζ-dependent target gene expression in IL-36α-stimulated HaCaT cells (Fig. 3d). Thus, the inventors' data imply that CDK4/6 specifically cooperate with STAT3 in a kinase-dependent manner, leading to the expression of IκBζ in keratinocytes.

### CDK4/6 phosphorylate EZH2 to induce STAT3-mediated IκBζ expression

Next, the inventors explored the mechanism of how CDK4/6 regulate STAT3-mediated expression of IκBζ. Of note, in chromatin immunoprecipitation (ChIP) analyses CDK4/6 was found to localize to the *NFKBIZ* promoter region, which depended on the presence of STAT3 (Fig. 5a). *Vice versa,* knockdown of CDK6 abrogated the binding of STAT3 at the *NFKBIZ* promoter (Fig. 5b). The inventors reasoned that this interdependency was due to a CDK4/6-dependent regulation of STAT3 activity in keratinocytes. Accordingly, whereas the putative CDK-dependent phosphorylation site of STAT3 at threonine 727 (T727) remained unaffected, phosphorylation of STAT3 at tyrosine 705 (Y705), a pre-requisite for STAT3 activation, was completely absent in abemaciclib-treated or CDK4/6-deficient cells after stimulation with IL-36α (Fig. 6a and Fig. 6b). As CDK4/6 are not able to directly trigger Y705 STAT3 phosphorylation, the inventors assumed that CDK4/6-mediated activation of STAT3 might be exerted through an altered availability or activation of a cofactor needed for STAT3 activation in keratinocytes.

Previously, EZH2, a methyltransferase that directs H3K27me3 in conjunction with the PRC2 complex, was found to be important in the differentiation and function of keratinocytes. Moreover, it was revealed that EZH2 can methylate STAT3 at lysine 49, 140 or 180, thereby modulating STAT3 activity by affecting the subcellular localization or phosphorylation status of STAT3 at tyrosine 705. We hypothesized that CDK4/6 might phosphorylate EZH2 in keratinocytes, thus enabling EZH2-mediated methylation and activation of STAT3. Pulldown assays in HEK293T cells validated an interaction of CDK4 and CDK6 with EZH2 (Fig. 5c). In agreement, EZH2 inhibition by EPZ6438 or shRNA-mediated depletion of EZH2 inhibited STAT3 activation and induction of IκBζ in IL-36α- or IL-17A/TNFα-stimulated keratinocytes (Fig. 6c and Fig. 5d). Furthermore, pharmacological inhibition or depletion of EZH2 effectively prevented IκBζ-dependent target gene expression in IL-36α-treated keratinocytes (Fig. 5e). Thus, we hypothesized that CDK4/6 phosphorylates EZH2 in keratinocytes, thereby regulating EZH2-dependent activation of STAT3.

In primary human keratinocytes, expression of EZH2 itself was induced by IL-36α (Fig. 6d), in line with its previous identification as an NF-κB-regulated target gene. Of note, EZH2 harbors two potential CDK phosphorylation sites at threonine 345 and 487 (Fig. 5f), which were previously shown to be phosphorylatable by CDK1/2, thereby modifying EZH2 function. Indeed, phosphorylation of EZH2 at threonine 345 (T345), but not at threonine 487 (T487) was induced in IL-36α- or IL-17A/TNFα-treated keratinocytes, whereas abemaciclib treatment or CDK4/6 depletion completely abrogated this inducible EZH2 phosphorylation (Fig. 6d and Fig. 5g). Moreover, phosphorylated EZH2 (T345) preferentially interacted with STAT3 in HaCaT cells, whereas CDK4/6 inhibition did not only abrogate the phosphorylation of EZH2 but also its interaction with STAT3 (Fig. 6e).

These data suggest that CDK4/6-mediated phosphorylation of EZH2 at threonine 345 represents a regulatory switch, leading to the interaction of EZH2 with STAT3 and subsequent STAT3 activation. Accordingly, whereas wildtype EZH2 synergistically induced the expression of the *NFKBIZ* luciferase promoter in cooperation with CDK4/6 and STAT3, an EZH2 mutant lacking the CDK4/6-directed phosphorylation site (EZH2 T345A) abrogated CDK4/6- and STAT3-mediated *NFKBIZ* promoter-driven luciferase expression (Fig. 6f). Furthermore, transient expression of a phospho-mimicking EZH2 (T345D) version could override abemaciclib-mediated suppression of IκBζ induction and IκBζ target gene expression in IL-36α-stimulated HaCaT cells (Fig. 6g), whereas transient overexpression of IκBζ abolished the effects of the pharmacological EZH2 inhibitor (Fig. 6h). Finally, also STAT3C overexpression could override target gene expression defects in IL-36α-stimulated, EZH2-depleted keratinocytes (Fig. 5h), thereby validating STAT3 as the main target for suppression of gene expression in EZH2 inhibitor-treated keratinocytes. Therefore, the inventors conclude that IL-36α- and IL-17A/TNFα-mediated, CDK4/6-dependent induction of IκBζ expression is mediated by phosphorylation of EZH2 at T345, thereby triggering an EZH2-dependent activation of STAT3 in keratinocytes.

### CDK4/6-phosphorylated EZH2 mediates STAT3 methylation at K180 leading to IκBζ expression in keratinocytes

As reported before, EZH2 can methylate STAT3 at lysine K49, K140 or K180, thereby changing its transcription factor function or subcellular localization. Thus, the inventors immunoprecipitated STAT3 in STAT3- and EZH2-overexpressing HEK293T cells in the presence or absence of abemaciclib, and analyzed the methylation status of STAT3 using a pan-methyl-lysine-specific antibody. Simultaneous overexpression of EZH2 and STAT3 induced methylation of STAT3, as expected, whereas CDK4/6 inhibition abrogated lysine methylation of STAT3 (Fig. 7a). Furthermore, lysine methylation of STAT3 was detectable upon co-overexpression of CDK6 and STAT3, whereas pharmacological EZH2 inhibition abrogated STAT3 methylation (Fig. 7b). Thus, CDK4/6 might indeed trigger an EZH2-dependent methylation and activation of STAT3.

EZH2-dependent methylation sites of STAT3 at lysine 49, 140 and 180 were previously identified by mass spectrometric analyses in tumor cells. Thus, the inventors substituted all three lysine methylation sites with arginines and tested the STAT3 mutants for their potential in activating *NFKBIZ* luciferase promoter expression. Whereas mutation of STAT3 at K49 and K140 had no effect on the induction of *NFKBIZ* promoter expression, alone or in combination with CDK6 and EZH2 (Fig. 8a), mutation of lysine K180 (STAT3 K180R), abrogated STAT3-mediated *NFKBIZ* promoter activation (Fig. 7c). Thus, the inventors hypothesized that CDK4/6-activated EZH2 methylates STAT3 at lysine 180, which is needed to induce IκBζ expression in stimulated keratinocytes. In agreement, reconstitution of CRISPR/Cas9-generated STAT3 knockout keratinocytes with wildtype STAT3, but not with the STAT3 K180R mutant, fully recovered IκBζ and IκBζ target gene expression upon IL-36α or IL-17A/TNFα stimulation (Fig. 7d and Fig. 8b). This was due to failure of STAT3 K180R in translocalizing to the nucleus in IL-36α-treated keratinocytes (Fig. 8c), as observed before. Accordingly, mutant STAT3 K180R and EZH2 failed to bind to the *NFKBIZ* promoter region in IL-36α-stimulated keratinocytes anymore (Fig. 7e). Thus, whereas IL-36α stimulation triggered wildtype STAT3 binding to the *NFKBIZ* promoter region together with EZH2 and CDK4/6, inhibition of CDK4/6 (Abe) or EZH2 (EPZ) abrogated the recruitment of this multi-protein complex (Fig. 7f). These results therefore suggest that CDK4/6 phosphorylates EZH2 to induce EZH2-dependent K180 STAT3 methylation, leading to the recruitment of the heteromeric complex to the *NFKBIZ* promoter and subsequent induction of IκBζ and IκBζ target gene expression in keratinocytes.

### Human and murine psoriatic lesions are characterized by overexpression of cyclin D2, cyclin D3 and EZH2

The inventors' findings suggest that CDK4/6 mediates the phosphorylation of EZH2 in a cyclin D-dependent manner, leading to STAT3 activation and IκBζ expression. The inventors therefore investigated a potential relevance of this pathway in skin biopsies from psoriasis patients. Human psoriatic lesions, compared to non-psoriatic lesions or unaffected skin, were characterized by a marked transcriptional upregulation of *CCND2* and CCND3, encoding cyclin D2 and cyclin D3, respectively (Fig. 9a). In contrast, *CCND1* mRNA levels were decreased in lesional skin biopsies. This is in line with the inventors' previous observation (Fig. 4c and 4d) that cyclin D1, unlike cyclin D2 and cyclin D3, did not synergize with CDK4/6 and STAT3 in increasing *NFKBIZ* promoter activity or expression of *NFKBIZ* and IκBζ target genes. In addition, *EZH2* mRNA levels were significantly upregulated in human psoriatic skin lesions (Fig. 9b). Immunohistochemistry further demonstrated that on the protein level, human EZH2, which was only weakly expressed in normal skin, was strongly overexpressed in the basal cell compartment of psoriatic skin lesions, revealing a typical nuclear localization (Fig. 9c).

Next, the inventors ask if an upregulation of cyclin D2, cyclin D3 and EZH2 can be also detected in relevant psoriasis mouse models. In the standard model using the TLR7 agonist imiquimod (IMQ), psoriasis-like skin inflammation was triggered by daily application of an IMQ-containing cream on the ears for 6 days, while in a second model daily intradermal injections of IL-36α into the skin of mouse ears were employed for 5 consecutive days (for details see Figure 10). After 6 or 7 days of treatment not only skin inflammation (see Fig. 10), but also increased expression of *Ccnd2, Ccnd3* and *Ezh2* mRNA was detectable in both animal models (Fig. 9d and 9e). Thus, in addition to the previously demonstrated overexpression of IκBζ in psoriasis, a hyperactive cyclin D-CDK4/6 pathway and elevated EZH2 expression are evident in murine and human psoriatic skin lesions.

### Topical application of CDK4/6 or EZH2 inhibitors protects against experimental psoriasis in vivo

Due to the inventors' finding that CDK4/6 and EZH2 inhibitors suppressed psoriasis-related, pro-inflammatory gene expression downstream of IL-36α or IL-17A/TNFα, the inventors next investigated the potential of CDK4/6 and EZH2 inhibitors to block experimental psoriasis *in vivo.* Moreover, the inventors reasoned that topical application of both inhibitors would be sufficient, as the epidermis constitutes the main target for CDK4/6 and EZH2 inhibition. A pre-requisite for efficient take-up of small molecule inhibitors from the skin are hydrophobic features of these substances. Thus, the inventors selected more hydrophobic inhibitors, such as abemaciclib (for CDK4/6 inhibition) or CPI-169 (see Bradley, W.D., et al., EZH2 inhibitor efficacy in non-Hodgkin's lymphoma does not require suppression of H3K27 monomethylation, Chem Biol 21, 1463-1475 (2014)) (for EZH2 inhibition) that can more easily penetrate the outer skin barrier. Psoriasis-like skin inflammation was induced in the above-mentioned psoriasis model by daily application of an IMQ-containing cream on the ears of wildtype mice for 6 days, before animals were sacrificed and analyzed at day 7. Abemaciclib, CPI-169 and ethanol as vehicle control were daily applied on the ear skin in parallel to IMQ (Fig. 11a). Whereas IMQ-treated ears exerted ear thickening, along with keratinocyte hyperproliferation and immune cell infiltration, topical application of abemaciclib or CPI-169 completely suppressed IMQ-induced, psoriasis-like skin inflammation (Fig. 10a and 10b). Both inhibitors effectively penetrated the skin and inhibited CDK4/6 or EZH2, as detected by loss of pRB (for CDK4/6 inhibition) or H3K27me3 (for EZH2 inhibition) expression in the epidermis of treated mice (Fig. 10c). Moreover, abemaciclib treatment significantly suppressed the infiltration of neutrophils, macrophages and T cells in IMQ-treated mice (Fig. 10d), while topical application of the EZH2 inhibitor CPI-169 fully abrogated immune cell infiltration upon IMQ treatment (Fig. 10e). Of note, also the number of infiltrating plasmacytoid (pDC) and myeloid dendritic cells (mDC) was significantly suppressed by application of both inhibitors (Fig. 12b). Importantly, whereas IMQ treatment effectively induced IκBζ expression in the skin, along with phosphorylation of EZH2 at T345 and of STAT3 at Y705, topical administration of abemaciclib or CPI-169 completely abrogated these signaling events (Fig. 10f and Fig. 12c). As a positive control, stabilization of the CDK4/6 substrate FOXM1, as well as EZH2-directed H3K27 methylation were strongly reduced in either CDK4/6 or EZH2 inhibitor-treated mouse skin (Fig. 9f). Accordingly, expression of IκBζ target genes, such as *Cxcl2* and *Cxcl5,* and DC- and T-cell-derived cytokines, such as *II17a* or *II23a,* was significantly downregulated in IMQ- and abemaciclib- or IMQ- and CPI-169-treated skin (Fig. 12d).

Treatment of mice with the TLR7 agonist IMQ represents a standard mouse model for psoriasis, however, IMQ activates immune cells in the first instance, rather than an initial keratinocyte-derived pro-inflammatory response, as it is likely to happen in human psoriasis pathogenesis. Thus, the inventors additionally investigated the therapeutic effects of abemaciclib or CPI-169 in an IL-36-triggered psoriasis-like dermatitis mouse model (Fig. 12e). As previously reported, repeated intradermal injections of IL-36α into the skin of mouse ears induced ear swelling, keratinocyte hyperproliferation along with immune cell infiltration (Fig. 10g and 10h). As a control for drug penetration in the skin of IL-36-treated animals, effective inhibition of CDK4/6 and EZH2 methyltransferase activity was controlled by staining for pRB and H3K27me3, respectively (Fig. 12f). Similar to the IMQ mouse model, topical application of abemaciclib or CPI-169 effectively blocked keratinocyte hyperproliferation and immune cell infiltration (Fig. 10g and 10h). Moreover, both inhibitors suppressed IL-36-mediated expression of IκBζ, phosphorylation of EZH2 at T345 and activation of STAT3 (pSTAT3 Y705) in the skin of treated mouse ears (Fig. 10i). Accordingly, IκBζ target gene expression and key cytokine expression, such as *II17a* and *II23a,* were effectively blocked as well (Fig. 12g). Thus, inhibition of CDK4/6 or EZH2 in IMQ- or IL-36-mediated psoriasis, effectively prevented psoriasis induction *in vivo,* by suppressing STAT3-mediated induction of IκBζ expression and IκBζ target gene expression. In view of its increased activity in human psoriatic skin, inhibition of this pathway by topical application of CDK4/6 inhibitors could therefore provide a new therapeutic option for the treatment of psoriasis patients.

### 4. Discussion

CDK4/6 inhibitors have been developed for treatment of cancer patients in order to restrain hyperproliferation of tumor cells. Recently, it was found that CDK4/6 do not only control cell cycle progression by phosphorylation of RB, but also regulate immune cell differentiation and function. In this context, CDK4/6 have been implicated as transcriptional cofactors that activate a subset of NF-κB or STAT3 target genes. Based on the inventors' results in cultured keratinocytes, human skin biopsies and mouse models, the inventors propose repurposing of CDK4/6 inhibitors for psoriasis therapy (Fig. 11). Moreover, the inventors' results uncovered a new pathway involving CDK4/6-mediated phosphorylation of EZH2 and EZH2-dependent methylation and activation of STAT3, leading to the inducible expression of IκBζ and IκBζ-dependent target genes in keratinocytes.

NFKBIZ, the gene encoding IκBζ, has been identified as a new susceptibility locus in psoriasis; see Tsoi, L.C., et al., Enhanced meta-analysis and replication studies identify five new psoriasis susceptibility loci. Nat Commun 6, 7001 (2015). The inventors recently reported that IκBζ is overexpressed in human psoriatic lesions, whereas global IκBζ KO mice are completely protected against psoriasis-like skin inflammation in several psoriasis models; see Johansen et al. (2015; loc. cit.) and Müller et al. (2018; loc. cit.). Mechanistically, IκBζ is transcriptionally induced in keratinocytes by IL-17 and IL-36, which triggers the expression of psoriasis-relevant target genes encoding for selective chemo- and cytokines and antimicrobial proteins. Deficiency of IκBζ therefore prevents the recruitment of neutrophils and monocytes that are needed for skin inflammation. Collectively, the inventors' data suggest that interfering with IκBζ expression or function in keratinocytes might be a promising strategy for psoriasis therapy. As IκBζ is crucial for both IL-36 and IL-17 signaling, CDK4/6 inhibitors might be applicable for different subtypes of psoriasis.

Unfortunately, based on a lack for enzyme activity, direct pharmacological inhibition of IκBζ function remains difficult. The inventors therefore sought to block the transcriptional induction of IκBζ and identified small molecule inhibitors of CDK4/6 and EZH2 as potent suppressors of IκBζ expression in keratinocytes. CDK4/6 have been previously shown to modulate several immune-relevant transcription factors by both kinase-dependent and - independent mechanisms. In the present study, the inventors clearly demonstrate that STAT3-mediated IκBζ expression is kinase-dependent, as ATP-competitive CDK4/6 inhibitors, such abemaciclib or palbociclib, abolished IκBζ expression. Consistent with these findings, a hyperactive but not a dominant-negative version of CDK6 increased *NFKBIZ* promoter activity. Moreover, cyclin D2 and cyclin D3 elevated the expression of *NFKBIZ* and its target genes, supporting the need for CDK4/6 kinase activity.

Despite the requirement of the kinase activity, the involvement of CDK4/6 could be separated from its classical role in cell cycle regulation and phosphorylation of RB. Thus, depletion of RB did not affect IκBζ expression. Moreover, IκBζ expression was principally induced by IL-36 stimulation in all phases of the cell cycle, except for G₀-arrested cells that revealed a weaker IκBζ expression. Importantly, although IκBζ expression does not rely on CDK4/6-mediated cell cycle progression, CDK4/6 inhibitors might have also beneficial effect in psoriasis treatment by additionally blocking the keratinocyte hyperproliferation, which is a hallmark of psoriasis.

In this study, the inventors demonstrate a major role for STAT3 in driving keratinocyte-specific IκBζ expression. IκBζ expression in keratinocytes is predominantly controlled from the proximal promoter 2 of the *NFKBIZ* locus, containing different transcription factor binding sites than the better investigated distal promotor 1, which is more tightly controlled by NF-κB. So far, the inventors have not compared the promoter usage in distinct cell types, but is likely that the contribution of the individual promoters and STAT3 to IκBζ expression differs among different cell types. The inventors' experiments show that CDK4/6 do not directly phosphorylate STAT3 but EZH2, which induces IκBζ and IκBζ-dependent pro-inflammatory target gene expression in a STAT3-dependent manner. This finding seems surprising at the first instance, since EZH2, as part of the PRC2 complex, is mainly involved in gene repression through trimethylation of H3K27. Recently, however, EZH2 was found to induce gene expression via interaction with the SWI/SNF complex or by repressing anti-inflammatory molecules, such as SOCS3, thus delimitating the expression of pro-inflammatory cytokines. CDK4/6 phosphorylated EZH2 at T345, thereby inducing an EZH2-dependent methylation of STAT3 at K180, and subsequent induction of IκBζ expression by STAT3. EZH2 phosphorylation at T345 was previously described to be mediated by CDK1 and CDK2, leading to an EZH2-directed epigenetic silencing of genes during G2 phase. Thus, even though CDK-mediated phosphorylation of EZH2 at T345 seems to be conserved, its impact on EZH2 function might depend on the stimulus or cell cycle phase.

Upon CDK4/6-mediated phosphorylation, EZH2 preferentially interacted with STAT3, resulting in STAT3 K180 methylation and enhanced STAT3 activation. Similar observations were made in glioblastoma, where IL-6-induced STAT3 activation is controlled by EZH2-mediated trimethylation of STAT3 at K180. Thus, phosphorylation of EZH2 might induce a switch in EZH2 function from H3K27 trimethylation and transcriptional repression to non-canonical functions, including STAT3 methylation and gene activation. Whether this gene-activating function of EZH2 requires the PRC2 repressor complex or whether it is PRC2-independent remains to be resolved. In addition to its main function in transcriptional repression, non-PRC functions of EZH2 via direct binding to transcriptional regulators have been reported before. For instance, EZH2 was shown to act as a co-factor for AR or the SWI/SNF complex leading to target gene activation. Similar to other non-histone targets, however, the exact molecular events that link STAT3 methylation to STAT3 activation are currently unknown.

Regardless of the detailed mechanism of EZH2-mediated STAT3 activation, the inventors' study has important clinical implications. The inventors' results suggest that targeting of the CDK4/6-EZH2-STAT3 pathway does not only suppress cytokine-mediated induction of IκBζ and pro-inflammatory target gene expression, but also inhibits immune cell recruitment and skin inflammation. The inventors demonstrate in the IMQ- and IL-36-mediated psoriasis mouse models that both CDK4/6 and EZH2 inhibitors completely blocked the development psoriatic skin lesions. The therapeutic effect of the inhibitors concurred with a suppression of IκBζ expression and a strong inhibition of IκBζ target gene expression, including chemokines (e.g. *Cxcl2, Cxcl5*)*,* cytokines (e.g. *II1f9, 111b, 1117a, II23a*) and antimicrobial proteins (e.g. *Lcn2).* In contrast, genes that were not IκBζ-dependent, such as *NFKBIA* and *TNF,* remained unaffected upon CDK4/6 or EZH2 inhibition. These findings further support the view of a rather selective role of IκBζ in the control of immune responses and also indicate that inhibition of IκBζ will be associated with less side effects than a broad inhibition of NF-κB by toxic IKK inhibitors.

In line with the inventors previous reports showing an upregulated expression of IκBζ in psoriasis (Johansen et al. (2015; loc. cit.) and Müller et al. (2018; loc. cit.), the inventors detected an increased nuclear expression of EZH2 and elevated cyclin D2 and D3 levels, both in psoriasis mouse models and in human psoriatic skin lesions. Previous studies also found constitutively active STAT3 in the epidermis of psoriatic lesions (see Miyoshi, K., et al., Stat3 as a therapeutic target for the treatment of psoriasis: a clinical feasibility study with STA-21, a Stat3 inhibitor, J Invest Dermatol 131, 108-117 (2011). The inventors assume that the CDK4/6-EZH2-STAT3 pathway is hyperactive in psoriatic skin lesions. As inhibition of IκBζ blocks multiple signaling pathways in psoriasis, targeting IκBζ might increase overall therapy responses as well as prevent the development of therapy resistance. Due to the clinical availability of hydrophobic CDK4/6 and EZH2 inhibitors, the inventors propose formulation of these inhibitors for instance in a crème, for topical treatment of psoriatic skin lesions. Topical drug administration will also restrict potential side effects and might be especially promising for those patients who have developed resistance to current psoriasis therapies.

## Claims

1. Abemaciclib for use in the treatment of psoriasis, wherein the use is via a topical application on the skin.

2. The CDK4/6 inhibitor for use according to any of the previous claims in combination with an additional agent active against psoriasis-associated symptoms.

3. The CDK4/6 inhibitor for use according to claim 2, wherein the additional agent is an EZH2 inhibitor.

4. A pharmaceutical composition for use in the treatment of psoriasis comprising abemaciclib and a pharmaceutically acceptable carrier, which is provided as a skin-permeable formulation, and wherein the use is via a topical application on the skin.

5. The pharmaceutical composition for use of claim 4, wherein the skin-permeable formulation is selected from the group consisting of: creme, gel, lotion.

6. The pharmaceutical composition for use of any of claims 4 to 5, wherein it comprises an additional agent active against psoriasis-associated symptoms.

7. The pharmaceutical composition for use of claim 6, wherein the additional agent is an EZH2 inhibitor.

## Patentansprüche

1. Abemaciclib zur Verwendung bei der Behandlung von Psoriasis, wobei die Verwendung über eine topische Applikation auf die Haut erfolgt.

2. CDK4/6-Inhibitor zur Verwendung gemäß einem der vorhergehenden Ansprüche in Kombination mit einem zusätzlichen Wirkstoff, der gegen Psoriasis-assoziierte Symptome wirksam ist.

3. CDK4/6-Inhibitor zur Verwendung gemäß Anspruch 2, wobei der zusätzliche Wirkstoff ein EZH2-Inhibitor ist.

4. Pharmazeutische Zusammensetzung zur Verwendung bei der Behandlung von Psoriasis, die Abemaciclib und einen pharmazeutisch akzeptablen Träger aufweist, und die als hautpermeable Formulierung bereitgestellt wird, und wobei die Verwendung über eine topische Applikation auf die Haut erfolgt.

5. Pharmazeutische Zusammensetzung zur Verwendung nach Anspruch 4, wobei die hautpermeable Formulierung ausgewählt ist aus der Gruppe bestehend aus: Creme, Gel, Lotion.

6. Pharmazeutische Zusammensetzung zur Verwendung nach einem der Ansprüche 4 bis 5, wobei sie einen zusätzlichen Wirkstoff aufweist, der gegen Psoriasis-assoziierte Symptome wirksam ist.

7. Pharmazeutische Zusammensetzung zur Verwendung nach Anspruch 6, wobei der zusätzliche Wirkstoff ein EZH2-Inhibitor ist.

## Revendications

1. Abémaciclib pour une utilisation dans le traitement du psoriasis, où l'utilisation se fait par une application topique sur la peau.

2. Inhibiteur de CDK4/6 pour une utilisation selon l'une des revendications précédentes en combinaison avec un agent supplémentaire actif contre les symptômes associés au psoriasis.

3. Inhibiteur de CDK4/6 pour une utilisation selon la revendication 2, dans lequel l'agent supplémentaire est un inhibiteur d'EZH2.

4. Composition pharmaceutique pour une utilisation dans le traitement du psoriasis, comprenant de l'abémaciclib et un support pharmaceutiquement acceptable, qui est fournie en tant que formulation perméable à travers la peau, et
dans laquelle l'utilisation se fait par une application topique sur la peau.

5. Composition pharmaceutique pour une utilisation selon la revendication 4, dans laquelle la formulation perméable à travers la peau est choisie dans le groupe constitué par : une crème, un gel, une lotion.

6. Composition pharmaceutique pour une utilisation selon l'une des revendications 4 et 5, dans laquelle elle comprend un agent supplémentaire actif contre les symptômes associés au psoriasis.

7. Composition pharmaceutique pour une utilisation selon la revendication 6, dans laquelle l'agent supplémentaire est un inhibiteur d'EZH2.
